(19) Europäisches Patentamt
European Patent Office
Office européen des brevets

(11) Numéro de publication : **0 028 959**
**B1**

(12) # FASCICULE DE BREVET EUROPÉEN

(45) Date de publication du fascicule du brevet : **30.01.85**

(21) Numéro de dépôt : **80401522.0**

(22) Date de dépôt : **27.10.80**

(51) Int. Cl.⁴ : **C 07 D455/03**, A 61 K 31/435// C07C47/575, C07C79/35, C07C93/14, C07C103/78, C07D217/20

(54) **Dérivés de la 5,8,13,13a-tétrahydro-6H-dibenzo (a, g) quinolizine, procédé d'obtention et compositions pharmaceutiques les contenant.**

(30) Priorité : **13.11.79 FR 7927989**

(43) Date de publication de la demande : **20.05.81 Bulletin 81/20**

(45) Mention de la délivrance du brevet : **30.01.85 Bulletin 85/05**

(84) Etats contractants désignés : **AT BE CH DE GB IT LI LU NL SE**

(56) Documents cités :
**GB-A- 1 004 077**
**Le dossier contient des informations techniques présentées postérieurement au dépôt de la demande et ne figurant pas dans le présent fascicule.**

(73) Titulaire : **U.R.P.H.A.**
**34, Boulevard de Clichy**
**F-75018 Paris (FR)**

(72) Inventeur : **Stambach, Jean-François**
**39, rue Wimpheling**
**F-67000 Strasbourg (FR)**
Inventeur : **Jung, Louis**
**205, Route d'Oberhaustergen**
**F-67200 Strasbourg (FR)**
Inventeur : **Schott, Claire Née Ensminger**
**7, Square du Chateau**
**F-67300 Schiltigheim (FR)**
Inventeur : **Heitz, Christiane Née Hummel**
**8, Rue de Zinsel**
**F-67300 Schiltigheim (FR)**
Inventeur : **Stoclet, Jean-Claude**
**13, Boulevard Sébastien Bach**
**F-67000 Strasbourg (FR)**

(74) Mandataire : **Phélip, Bruno et al**
**c/o Cabinet Harlé & Phélip 21, rue de la Rochefoucauld**
**F-75009 Paris (FR)**

**EP 0 028 959 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne des nouveaux dérivés de la 5,8,13,13a-tétrahydro-6H-dibenzo [a, g] quinolizine ; elle concerne également la synthèse desdits dérivés. L'invention a encore pour objet l'application thérapeutique de ces nouveaux dérivés et les compositions pharmaceutiques les contenant.

La 5,8,13,13a-tétrahydro-6H-dibenzo [a, g] quinolizine, dont la dénomination commune française est « Berbine » répond à la formule I ci-après :

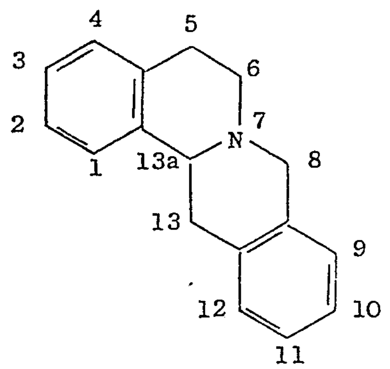

(I)

La « Berbine » inverse les effets hypertenseurs de l'adrénaline chez le chien et inhibe la contraction du muscle lisse provoquée par l'ion $K^+$ [Raymond Hamet, Bulletin de l'Académie Nationale de Médecine 1952, *22* et *23*, 408 ; Raymond Hamet C.R. Acad. Sci. 1953, *236*, 1616]. Elle exerce une action hypotensive chez des patients hypertendus [Faquet, Lisle et Combaz, Gazette Médicale de France, 1954, *61*, 1615].

D'autre part, le brevet GB 1 004 077 concerne des compositions contenant des dibenzoquinolizines qui possèdent une activité tranquillisante, antidépressive et antiémétique.

Les dibenzoquinolizines, contenues dans ces compositions pharmaceutiques à titre d'ingrédient actif, répondent à la formule générale ci-après :

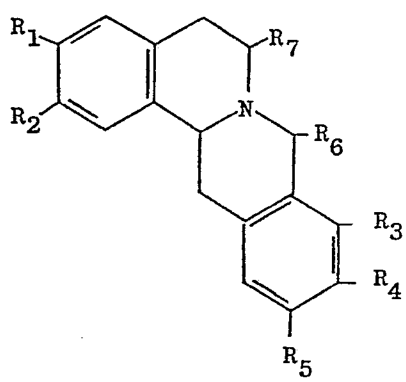

dans laquelle :

$R_1$ et $R_2$ représentent notamment l'hydrogène, les groupes hydroxy, méthoxy ou éthoxy ;

$R_3$ et $R_5$ représentent l'hydrogène ou le groupe méthoxy ;

$R_4$ représente l'hydrogène, les groupes hydroxy, méthoxy ou éthoxy et

$R_6$ et $R_7$ représentent l'hydrogène ou le groupe méthyle.

Cependant les enseignements de ce brevet GB 1 004 077 ne permettaient pas d'atteindre les dérivés hydroxylés en position 3.

En effet, les conditions opératoires du procédé mentionné à la page 2, lignes 59 à 64 de ce brevet GB 1 004 077 se révèlent être trop drastiques (acidité et chaleur) pour pouvoir conserver un groupe benzyloxy (protecteur de la fonction hydroxy) au cours de la cyclisation des phénylacétamides N-substitués en 3,4-dihydro-isoquinoléines, réalisée en présence d'oxychlorure de phosphore et par chauffage.

On a maintenant synthétisé des nouveaux dérivés de la « Berbine » qui présentent une activité pharmacologique intéressante.

Les dérivés selon la présente invention répondent à la formule générale II :

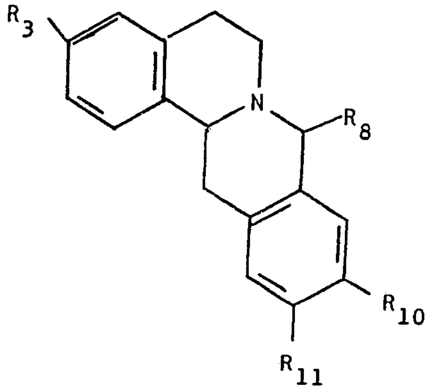

(II)

dans laquelle :

$R_3$ est un radical —OR ou —SR, R étant l'hydrogène, un groupe alkyle en $C_1$ à $C_{12}$ ou phényle ou phénylalkyle (alkyle en $C_1$ à $C_4$) ou un groupe de formule

$$R'-\underset{\underset{O}{\|}}{C}-\ ,$$

R' étant un groupe alkyle en $C_1$ à $C_{12}$ ou phénylalkyle (alkyle en $C_1$ à $C_4$)

$$R_8 \text{ est } = O \text{ ou } \begin{matrix} \diagup H \\ \diagdown H \end{matrix}$$

$R_{10}$ et $R_{11}$, identiques ou différents, représentent l'hydrogène ou un groupe alcoxy inférieur en $C_1$ à $C_4$ à la condition que, lorsque $R_8$ est $\begin{matrix} \diagup H \\ \diagdown H \end{matrix}$

$R_{10}$ et $R_{11}$ sont l'hydrogène ou un groupe alcoxy inférieur, $R_3$ est différent du groupe OR dans lequel R est le groupe alkyle.

Dans la présente description, le terme « alkyle » désigne des groupes hydrocarbonés aliphatiques contenant 1 à 12 atomes de carbone, à chaîne droite ou ramifiée. On préfère les groupes alkyle inférieurs, c'est-à-dire les groupes alkyle contenant 1 à 4 atomes de carbone.

Les composés particulièrement préférés selon l'invention sont ceux qui répondent aux formules IIa et IIb ci-après :

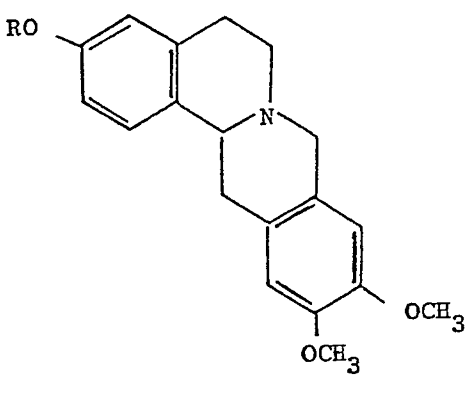

(IIa)

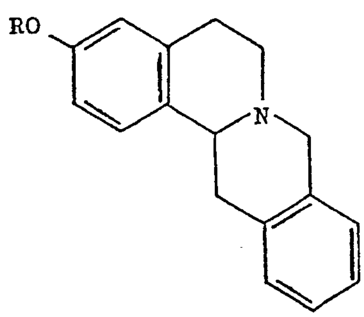

(IIb)

dans lesquelles R est l'hydrogène ou le groupe

$$R'-\underset{\underset{O}{\|}}{C}-$$

avec R' choisi parmi les groupes —$CH_3$ ; —$CH_2CH_3$ ; —$(CH_2)_2$—$CH_3$ ; —$C_6H_5$ ; —$CH_2$—$C_6H_5$.

L'invention concerne également la synthèse de ces nouveaux dérivés.

Sous son aspect général, le procédé de synthèse selon la présente invention consiste à :

1. condenser, avec du nitrométhane, le benzaldéhyde substitué en position 3 par un groupe $R_3$ protégé, pour former le nitrostyrène correspondant ;

2. réduire le nitrostyrène obtenu pour former la phénéthylamine correspondante ;

3. condenser la phénéthylamine obtenue avec l'acide phénylacétique substitué de formule

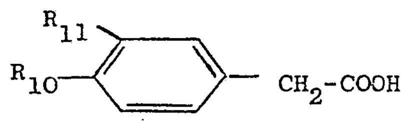

pour former le phénacétamide correspondant :

4. procéder à une première cyclisation pour obtenir la 3,4-dihydroisoquinoléine correspondante ;

5. réduire la dihydroisoquinoléine en tétrahydroisoquinoléine ;

6. fixer, par condensation avec de l'acide formique ou du phosgène, un groupe formyle ou chloroformyle sur l'atome d'azote de l'isoquinoléine ;

7. procéder à une seconde cyclisation ;

8. éventuellement réduire le produit résultant pour former le dérivé de « Berbine » désiré ;

9. éventuellement, éliminer le groupement protecteur en position 3 ; et

10. éventuellement, estérifier le produit résultant.

Comme cela est indiqué ci-dessus, le procédé de synthèse des composés de l'invention comporte deux variantes au niveau de l'étape 6 ; l'une des variantes consiste en la formylation de la tétrahydroisoquinoléine obtenue à l'étape 5 et l'autre consiste en la chloroformylation de ladite tétrahydroisoquinoléine qui permet l'obtention des composés de formule II dans laquelle $R_8$ est l'oxygène, les radicaux $R_3$, $R_{10}$ et $R_{11}$ étant tels que définis précédemment.

Le procédé de l'invention va être maintenant décrit en détail en référence aux schémas réactionnels 1 et 2 ci-après, qui représentent les schémas réactionnels des deux variantes du procédé mentionnées ci-dessus.

Le produit de départ mis en œuvre selon le procédé de l'invention est un benzaldéhyde substitué en 3 par un radical $R_3$ protégé, c'est-à-dire que $R_3$ doit être différent des groupes OH et SH libres. Cependant, on peut utiliser comme produit de départ des benzaldéhydes substitués en position 3 par un groupe OH ou SH, auquel cas ces composés doivent être soumis, avant l'étape (1) du procédé de l'invention, à une étape préliminaire qui consiste à protéger la fonction OH ou SH. Cette étape peut être réalisée selon des moyens bien connus de l'homme de l'art. On indiquera par exemple que cette réaction peut être réalisée par condensation du 3-hydroxy-benzaldéhyde avec un chlorure, tel que le chlorure de benzyle, en présence d'éthylate de sodium à reflux en milieu anhydre.

L'étape (1) du procédé de l'invention consiste à condenser le benzaldéhyde de formule I avec du nitrométhane $CH_3—NO_2$ pour former le nitrostyrène correspondant de formule 2. Cette réaction doit être réalisée en milieu acide anhydre en présence d'un agent de condensation approprié, tel que l'acétate d'ammonium, et à la température de reflux. On opère avantageusement en milieu acide acétique anhydre.

L'étape (2) du procédé de l'invention consiste à réduire le nitrostyrène de formule 2 en l'amine correspondante de formule 3 en présence d'un agent réducteur approprié, tel que l'hydrure de lithium et d'aluminium. Cette réaction de réduction est avantageusement réalisée dans un solvant inerte, tel que par exemple le tétrahydrofuranne, à la température ambiante ;

L'étape (3) du procédé consiste à condenser l'amine de formule 3 avec l'acide phénylacétique de formule :

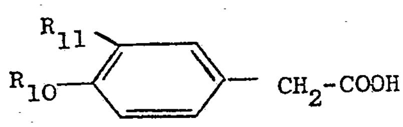

pour former le phénacétamide de formule 4. Cette condensation est avantageusement réalisée par fusion directe vers 200 °C de l'acide phénylacétique et de l'amine de formule 3 en quantités sensiblement équimoléculaires.

On obtient, après déshydratation du sel, l'amide de condensation de formule 4, c'est-à-dire le N-phénéthylphénacétamide substitué en position 3 par le radical $R_3$.

L'étape (4) du procédé de synthèse selon l'invention est une cyclisation qui est réalisée en présence d'un agent de cyclisation, tel que par exemple le pentachlorure de phosphore, dans un solvant, par exemple le chloroforme. Cette réaction est avantageusement réalisée à la température ambiante.

(Voir Schémas pages suivantes)

Schéma réactionnel 1

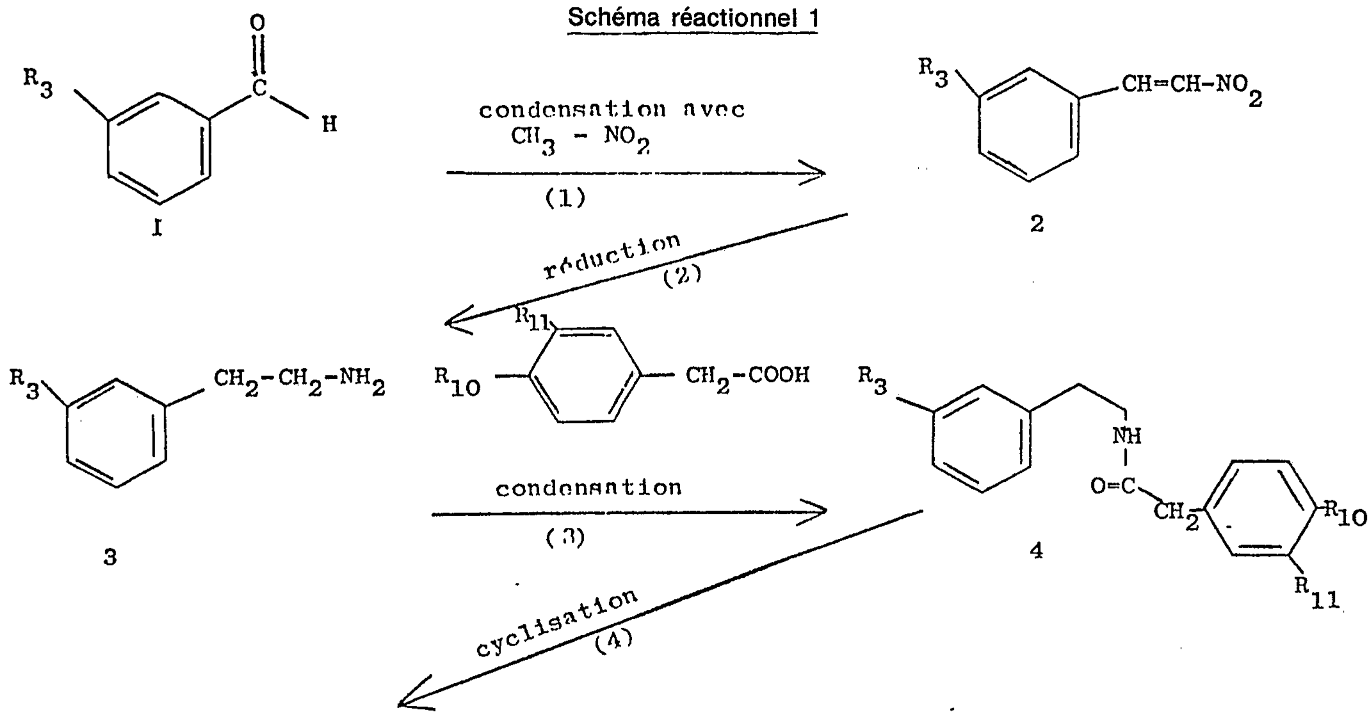


I

2

3

4

Schéma réactionnel 1 (suite)

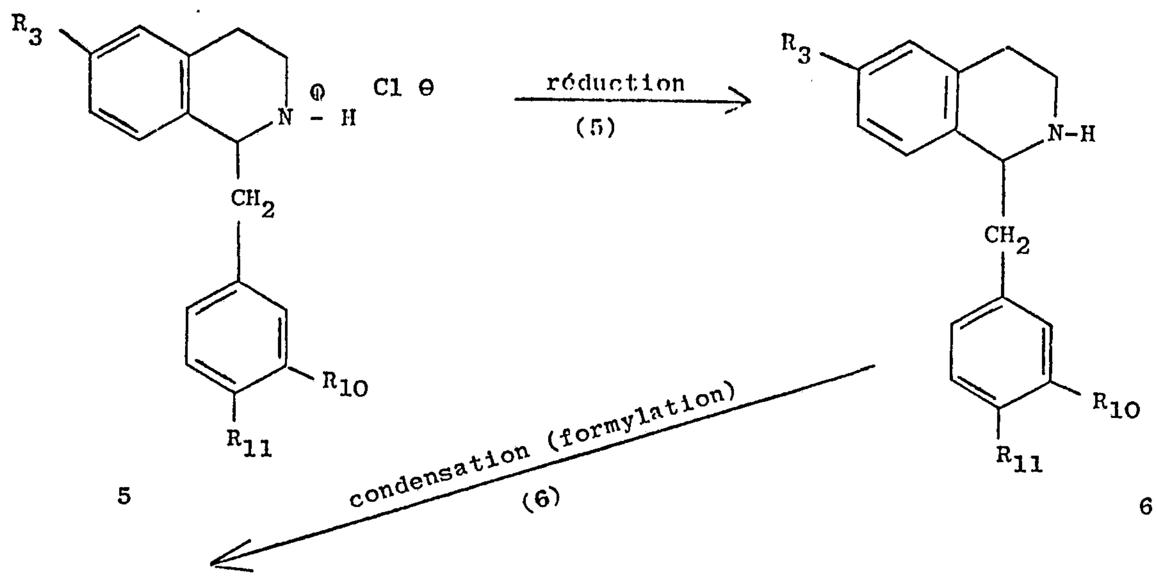


5

6

Schéma réactionnel 1 (suite)

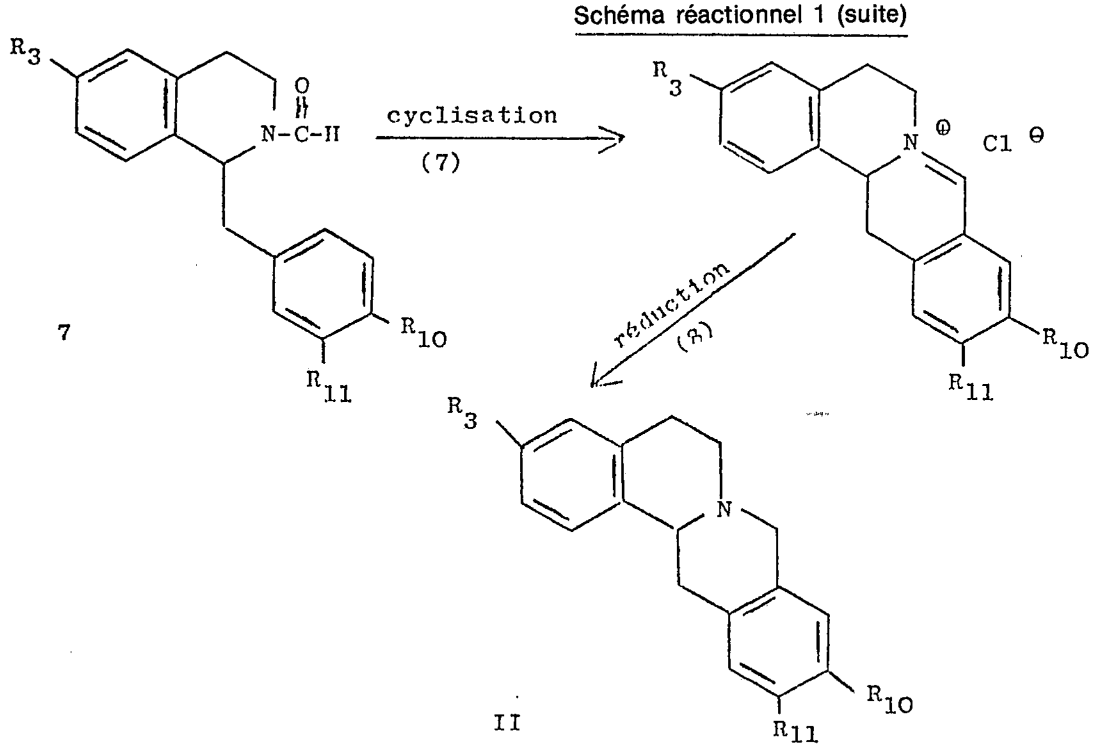


7

8

II

On obtient ainsi la 1-benzyl-3,4-dihydroisiquinoléine de formule 5 sous la forme de son chlorhydrate.

L'étape (5) du procédé de l'invention consiste en une réduction de la dihydroisoquinoléine de formule 5 en la tétrahydroisoquinoléine correspondante (formule 6). Cette réduction est réalisée en présence d'un catalyseur de réduction approprié tel que le zinc, dans un solvant, par exemple l'acide acétique dilué.

Selon la variante représentée sur la figure 1, l'étape (6) du procédé est une étape de formylation au cours de laquelle on fixe sur l'atome d'azote de la tétrahydroisoquinoléine un groupe formyle. Cette formylation est réalisée par condensation du composé de formule 6 avec de l'acide formique. Cette réaction est réalisée par fusion du mélange acide formique/tétrahydroisoquinoléine.

L'étape (7) du procédé consiste en une seconde cyclisation. Comme la première cyclisation, celle-ci est réalisée en présence d'un agent de cyclisation, tel que le pentachlorure de phosphore, dans un solvant approprié, par exemple le chloroforme. Cette réaction est avantageusement réalisée à la température ambiante. Il se produit une déshydratation interne aboutissant à une structure berbénique déshydrogénée aux positions 7 et 8 (composé de formule 8).

L'étape (8) du procédé de l'invention consiste en une réduction du composé de formule 8, et plus précisément en une réduction de la double liaison en 7,8 du composé de formule 8. Cette réduction peut être réalisée en présence du zinc, comme agent réducteur, en milieu acide.

Lorsqu'on opère en milieu acide faible, par exemple en milieu acide acétique, il se produit uniquement la réduction de la double liaison en 7,8. Par contre, lorsqu'on opère en milieu acide fort, par exemple en milieu acide chlorhydrique ou acide bromhydrique, il se produit également une hydrolyse du groupe protecteur du radical $R_3$.

Ainsi, les étapes 8 et 9 peuvent être partiellement combinées ou non.

Ensuite, lorsqu'on a obtenu le composé de formule II, dans laquelle $R_3$ est OH ou SH, $R_8$ est 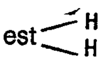

on peut former, si on le désire, les esters correspondants selon les modes opératoires d'estérification bien connus de l'homme de l'art (étape 10).

On indiquera par exemple que lorsque $R_3$ est le groupe OH on peut obtenir les esters correspondants par réaction dudit composé de formule II avec un chlorure d'acide de formule

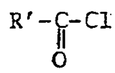

en présence d'une base, telle que la triéthylamine, dans un solvant approprié, tel que le chloroforme. Après extraction de l'ester formé, celui-ci est recristallisé dans un solvant approprié ; le produit ainsi obtenu répond à la formule II dans laquelle $R_3$ est le radical

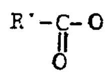

Selon une variante de mise en œuvre du procédé de l'invention représentée par le schéma réactionnel 2 ci-après, on opère à partir du composé de formule 6 (obtenu selon les étapes 1 à 5 du procédé de l'invention illustrées par le schéma réactionnel 1 et décrites en détails ci-dessus), une chloroformylation avec du phosgène en milieu basique anhydre en présence de triéthylamine dans un solvant approprié, tel que le chloroforme. On obtient ainsi la 1-benzyl-2-chloroformyl-1,2,3,4-tétrahydroisoquinoléine de formule 7a.

On procède ensuite à la cyclisation du produit chloroformylé de formule 7a en présence d'un catalyseur de cyclisation approprié, tel que le chlorure d'aluminium anhydre ou le chlorure de zinc anhydre. On opère avantageusement dans le benzène à reflux.

On obtient ainsi la 8-berbinone de formule 8a, c'est-à-dire le composé de formule II dans laquelle $R_8$ est un oxygène carbonylique. Ces composés de formule II avec $R_8$ étant un oxygène carbonylique sont notamment des intermédiaires de valeur pour l'obtention des composés de formule II avec $R_8$ = H, qui est réalisée (étape 8a) par réduction en présence d'un catalyseur approprié, tel que l'hydrure de lithium et d'aluminium, dans un solvant, par exemple le tétrahydrofuranne. On obtient ainsi le composé de formule II dans laquelle $R_8$ est 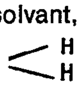

(Voir Schéma page 9)

Schéma réactionnel 2

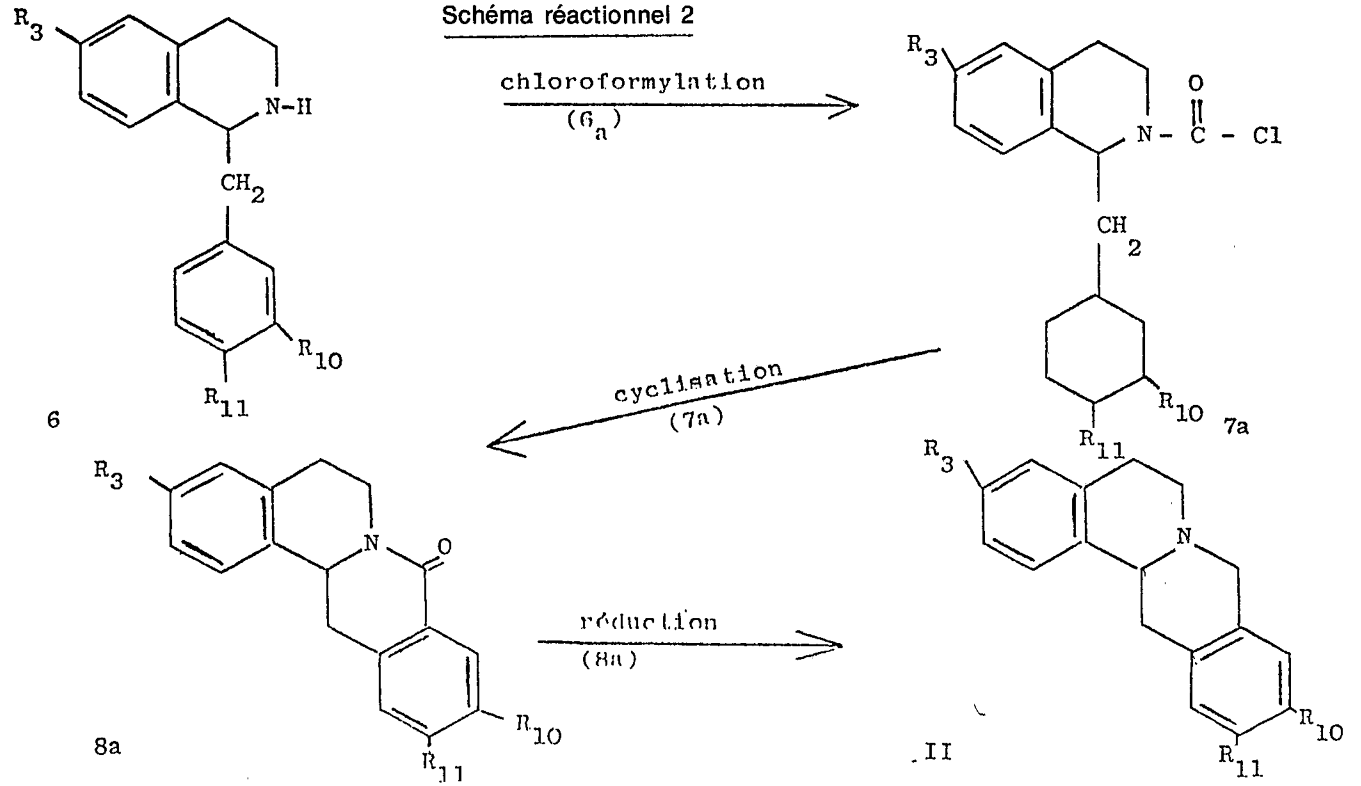


6

7a

8a

II

Le composé de formule II ainsi obtenu est ensuite éventuellement hydrolysé en milieu acide fort, par exemple en milieu acide chlorhydrique ou bromhydrique, pour éliminer le groupe protecteur du radical $R_3$.

Les composés de formule II ainsi obtenus sont ensuite éventuellement estérifiés selon le mode opératoire décrit précédemment.

Les composés selon l'invention possèdent des propriétés pharmacologiques intéressantes, notamment dans le domaine cardio-vasculaire pour lutter contre les troubles du rythme et les insuffisances cardiaques et coronariennes.

La présente invention concerne donc également les compositions pharmaceutiques contenant, à titre d'ingrédient actif, un composé de formule II en combinaison avec un véhicule approprié. Les compositions pharmaceutiques selon l'invention peuvent se présenter notamment sous des formes appropriées pour une administration par voie orale ou parentérale.

Pour une administration par voie orale, on pourra utiliser les compositions de l'invention sous la forme de comprimés, de gélules, de sirops, de gouttes, les véhicules utilisés étant des véhicules classiques pour la formation de ces formes pharmaceutiques et inertes vis-à-vis de l'ingrédient actif.

L'invention va maintenant être décrite plus en détail dans les exemples ci-après.

Exemple 1

Préparation de la 3-hydroxy-10,11-diméthoxy-5,8,13,13a-tétrahydro-6H-dibenzo [a, g] quinolizine (composé de formule II avec $R_3$ = OH, $R_8$ = H, $R_{10}$ et $R_{11}$ = $CH_3O$—)

a) Etape préliminaire : 3-benzyloxy-benzaldéhyde (composé de formule 1).

Dans un ballon de 1 l muni d'un réfrigérant, surmonté d'un tube à $CaCl_2$, et d'un agitateur mécanique, on a introduit 400 cc d'éthanol absolu et 24 g de sodium métallique avec précaution. Après que tout le sodium a réagi, on a dissous 125 g de 3-hydroxy-benzaldéhyde et additionné 135 cc de chlorure de benzyle. On a ensuite porté le mélange réactionnel à reflux, en agitant, au bain de glycérine (90-100 °C) pendant 4 heures. Puis on a distillé la plus grande partie de l'alcool au rotavapor ; on a repris le résidu par de l'eau et on l'a extrait à l'éther. On a lavé la phase éthérée à la soude diluée puis à l'eau. On a séché cette phase sur sulfate de magnésium, on l'a filtrée, on a évaporé l'éther au rotavapor, puis procédé à une distillation sous vide (12-15 mm de Hg) du chlorure de benzyle en excès (98-102 °C), puis du produit attendu vers 208-212 °C.

On a obtenu 170 g (rendement 78 %) du 3-benzyloxy-benzaldéhyde ; point de fusion 55-56 °C.

Tous les points de fusion indiqués dans la présente description ont été mesurés sur un appareil automatique METTLER FP 61.

b) étape (1) : 3-benzyloxy-nitrostyrène (composé de formule 2).

Dans un ballon de 3 l muni d'un réfrigérant, on a dissous les 170 g de 3-benzyloxy-benzaldéhyde dans 1 000 cc d'acide acétique glacial, et on a rajouté ensuite

15 cc d'anhydride acétique,
270 cc de nitrométhane et
102 g d'acétate d'ammonium sec.

Le mélange ainsi obtenu a été porté au reflux pendant 1 h 30, puis on a ajouté 100 cc d'$H_2O$ au mélange encore chaud. On a laissé le mélange réactionnel refroidir, après quoi le nitrostyrène a précipité en belles aiguilles jaunes dans le milieu. On a récupéré le précipité par filtration, on l'a lavé avec un mélange eau/acide acétique (15/85) puis avec de l'eau. On l'a ensuite séché.

On a obtenu 142 g de nitrostyrène (rendement 70 % ; point de fusion 91 °C : METTLER FP 61). Le produit a pu être utilisé tel quel pour la suite.

c) étape (2) : 3-benzyloxy-phénéthylamine (composé de formule 3).

Dans un tricol de 5 l équipé d'un réfrigérant, d'un thermomètre, d'une ampoule à brome et d'une agitation mécanique, on a introduit :

57 g de $LiAlH_4$,
2 000 cc d'éther anhydre (sur Na).

On a ensuite ajouté, goutte à goutte, à l'aide d'une ampoule à brome, la solution suivante :

124 g de 3-benzyloxy-nitrostyrène (composé de formule 2),
2 250 cc de THF absolu.

Après addition (6 à 8 heures), on a laissé les produits réactionnels en contact encore 1 heure, puis on a hydrolysé l'excès d'hydrure de la façon suivante : on a rajouté dans l'ordre, en refroidissant le ballon dans un bain de glace :

57 cc d'eau,
57 cc de NaOH à 15 %,
171 cc d'eau.

On a filtré les hydroxydes formés, on les a élués et essorés, puis on a évaporé le filtrat au rotavapor. Il restait une huile jaune à partir de laquelle on a distillé l'amine attendue sous 3-5 mm Hg vers 200 °C.

On a obtenu 95 g de phénéthylamine de formule 3 (rendement 75 %).

d) étape (3) : N-(3-benzyloxy-phénéthyl)-3,4-diméthoxy-phénacétamide (composé de formule 4).

On a condensé les 95 g de phénéthylamine obtenue ci-dessus mole à mole avec 82,1 g d'acide 3,4-diméthoxy-phénylacétique, par fusion entre 190-210 °C pendant 3-4 heures (jusqu'à l'arrêt de l'effervescence). Pour cela, on a introduit les deux composés dans un ballon de 500 $cm^3$ surmonté d'un tube de verre pour le dégagement de la vapeur d'eau qui se forme au cours de la réaction. On a plongé le ballon dans un bain d'alliage de Wood en maintenant la température aux alentours de 200 °C.

Après l'arrêt de l'effervescence, on a coulé la masse encore chaude dans un mortier et on l'a laissé refroidir. L'amide attendu a cristallisé. On a pulvérisé l'amide dans le mortier et on l'a lavé à l'éther pour enlever la coloration jaune. On a obtenu 161 g du composé attendu.

Point de fusion : 98 °C ;
rendement : 95 %.

e) étape (4) : [3,4-diméthoxy-benzyl] 6-benzyloxy-3,4-dihydroisoquinoléine (composé de formule 5)

Dans un erlenmeyer de 3 litres, on a introduit l'amide obtenu ci-dessus (161 g) et 1 150 cc de chloroforme pour le dissoudre. On a ensuite ajouté, sous agitation et par petites quantités, 215 g de pentachlorure de phosphore bien pulvérisé. On a maintenu l'agitation mécanique constante à 20 °C. Après environ 3/4 h à 1 heure, le chlorhydrate du produit attendu a précipité dans le milieu réactionnel. On a laissé les produits en contact pendant 2 heures en tout. Puis, on a fait précipiter totalement le chlorhydrate par addition de 1 150 $cm^3$ d'éther de pétrole léger (40-60 °C). On a filtré sur verre fritté le précipité et on l'a additionné rapidement et avec précaution (effervescence) à 300 $cm^3$ d'éthanol à 95 °C dans un bécher de 5 litres. Tout le produit s'est dissous, au besoin par chauffage. On a ensuite ajouté rapidement, sous agitation vigoureuse, 1,5 litre d'éther. Le chlorhydrate a reprécipité abondamment. On l'a lavé avec un mélange d'éthanol 95°/éther (3/7), on l'a filtré et séché. On a obtenu ainsi 126 g du composé de formule 5 sous la forme de son chlorhydrate (70-80 %) point de fusion 131-132 °C.

f) étape (5) : 1-[3,4-diméthoxy-benzyl] 6-benzyloxy 1,2,3,4-isoquinoléine (composé de formule 6).

On a dissous les 126 g de chlorhydrate du composé obtenu précédemment dans 1,5 litre d'acide acétique et on a ajouté 250 cc d'eau puis 136 g de zinc en poudre, le tout dans un ballon de 5 litres muni d'un réfrigérant. On a porté à reflux au bain de glycérine pendant 4 heures sous constante agitation. Puis, on a filtré l'excès de zinc à chaud et évaporé le filtrat au rotavapor. On a extrait à l'aide de chloroforme l'amine attendue en rajoutant au résidu eau et ammoniaque concentrée en large excès. On a lavé les phases chloroformiques à l'eau puis on les a séchées sur sulfate de magnésium. On a filtré et évaporé le chloroforme. On a obtenu 111 g de résidu sirupeux que l'on a utilisé tel quel dans la suite.

g) étape (6) : 1-[3,4-diméthoxy-benzyl] 6-benzyloxy-2-formyl-1,2,3,4-tétrahydroisoquinoléine (composé de formule 7)

On a introduit les 111 g de tétrahydroisoquinoléine obtenue précédemment dans un ballon de 500 $cm^3$ et on les a dissous dans 150 cc d'acide formique pur. On a plongé le ballon dans l'alliage de Wood et augmenté progressivement la température. L'excès d'acide formique a distillé vers 100-105 °C. On a maintenu alors la température aux alentours de 200 °C. Il s'est produit une effervescence avec dégagement de vapeur d'eau. Après la fin de l'effervescence (4-5 heures) on a coulé la masse encore chaude dans un mortier. On a laissé refroidir puis pulvérisé le produit formylé attendu et on l'a lavé à l'éther et séché. On a obtenu 116 g de l'isoquinoléine de formule 7. Rendement : 98 %-point de fusion : 131-132 °C.

h) étape (7) : chlorure de 3-benzyloxy-10,11-di-méthoxy-5,6,13,13a-tétrahydro-dibenzo-[a, g] quinolizinium (composé de formule 8).

On a dissous dans un erlenmeyer de 3 l les 116 g du produit formulé ci-dessus dans 1 litre de chloroforme R.P. On a ajouté en plusieurs fractions 150 g de pentachlorure de phosphore bien pulvérisés. Tout le pentachlorure s'est dissous. Après 10 à 15 minutes d'agitation constante à 20 °C, le produit a précipité. On a laissé en contact 1/2 h en tout. Puis on a complété la précipitation par addition de 1 l d'éther de pétrole léger (40-60 °C). On a filtré le produit sur verre fritté et on l'a bien égoutté. Puis on l'a additionné rapidement à de l'éthanol absolu (légère effervescence) et dissous à chaud dans l'éthanol. Le produit a reprécipité à froid. On l'a filtré, lavé à l'alcool à 95 °C et séché. On a obtenu ainsi 98 g du chlorure ci-dessus [rendement 80 %], point de fusion : 190-191 °C (décomposition).

i) étape (8) : 3-hydroxy-10,11-diméthoxy-5,8,13,13a-tétrahydro-6H-dibenzo [a, g] quinolizine (composé de formule II avec $R_3 = OH$, $R_8 = H$, $R_{10}$ et $R_{11} = CH_3—O—$).

Dans un ballon de 2 l muni d'un réfrigérant et d'une agitation mécanique, on a dissous les 98 g du chlorure obtenu ci-dessus dans :

800 cc d'acide acétique,
200 cc d'acide chlorhydrique concentré,
100 cc d'eau.

On a ajouté 150 g de zinc en poudre et le mélange réactionnel a été porté à ébullition au bain de glycérine ; on a laissé les produits en contact pendant 2 heures à reflux puis on a filtré l'excès de zinc à chaud.

On a évaporé le filtrat au rotavapor. On a repris le résidu par de l'eau, de l'ammoniaque concentrée en large excès et on a extrait au chloroforme. On a lavé la phase chloroformique. On l'a séchée sur du sulfate de magnésium. On a évaporéé le chloroforme au rotavapor. Il restait un précipité blanc du produit attendu. On a lavé ce précipité à l'éther, séché et recristallisé dans de l'alcool à 95°.

On a obtenu finalement 52 g (rendemernt 85 %) du composé de formule II, c'est-à-dire de la 3-hydroxy-10,11-diméthoxy-5,8,13,13a-tétrahydro-6H-dibenzo-[a, g]-quinolizine.

Le $R_f$ de la chromatographie en couche mince du produit obtenu est de 0,34 la chromatographie étant réalisée avec les solvants ci-après :

$CHCl_3$ : 25 cc
acétate d'éthyle : 25 cc ; point de fusion : 207 °C
triéthylamine : 1 cc

## Exemple 2

Préparation de la 3-benzyloxy-10,11-diméthoxy-5,8,13,13a-tétrahydro-6H-dibenzo [a, g] quinolizine

On a opéré selon le mode opératoire décrit à l'exemple 1 jusqu'à l'obtention du chlorure de 3-benzyloxy-10,11-diméthoxy-5,6,13,13a-tétrahydrodibenzo [a, g] quinolizinium (composé de formule 8). Ensuite, on a réduit sélectivement la double liaison dudit chlorure par dissolution dans de l'acide acétique dilué et en présence de zinc à reflux 3 heures. On a extrait le composé non débenzylé en 3, c'est-à-dire la 3-benzyloxy-10,11-diméthoxy-5,8,13,13a-tétrahydro-6H-dibenzo [a, g] quinolizine (composé 10) et on l'a recristallisé dans le méthanol (rendement 75 %).

On a ensuite débenzylé le groupe $R_3$ dudit composé ainsi obtenu par hydrolyse en présence d'acide chlorhydrique à 20 % et d'acide acétique dilué et par chauffage, et on a obtenu la 3-hydroxy-10,11-diméthoxy-5,8,13,13a-tétrahydro-6H-dibenzo [a, g] quinolizine. Le schéma réactionnel de ce mode opératoire, qui illustre le fait que les étapes 8 et 9 peuvent être effectuées séparément, est représenté ci-après :

(Voir Schéma page 13)

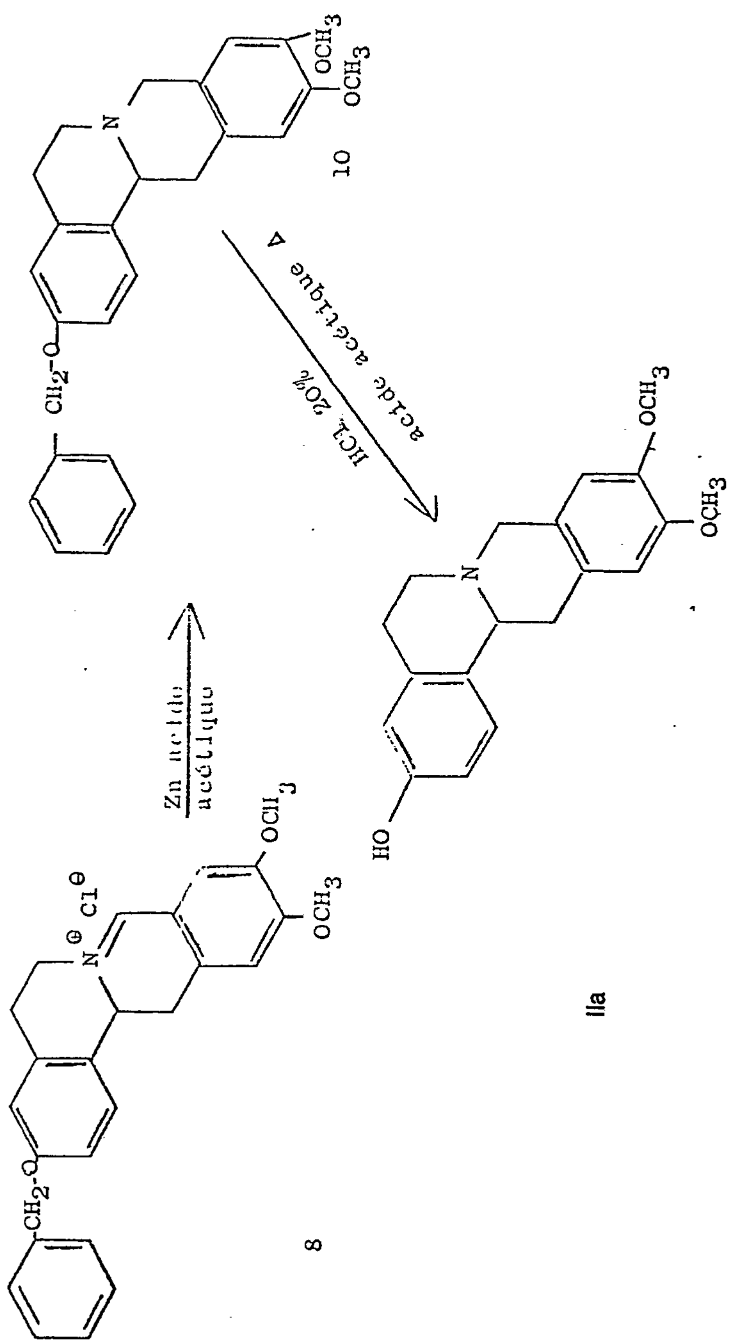


10

8

IIa

Exemple 3

Variante du procédé de l'invention : chloroformylation pour l'obtention de la 3-hydroxy-10,11-diméthoxy-5,8,13,13a-tétrahydro-6H-dibenzo [a, g]-quinolizine

Cet exemple illustre le procédé de l'invention comportant une étape de chloroformylation [schéma réactionnel 2].

On a utilisé le composé de formule 6 obtenu selon l'exemple 1 ci-dessus, c'est-à-dire le 1-[3,4-diméthoxybenzyl]-6-benzyloxy-1,2,3,4-tétrahydroisoquinoléine et on a opéré de la façon suivante :

a) étape (6a) : 3-benzyloxy-10,11-diméthoxy-2-chloroformyl-1,2,3,4-tétrahydroisoquinoléine.

On a équipé un tricol de 2 l d'une ampoule à brome, d'un réfrigérant, d'une arrivée de phosgène (30-40 ml/mn). On a introduit dans ce tricol :

300 cc de chloroforme R.P.

25 cc de triéthylamine.

On a fait barboter le phosgène, en agitant bien, pendant 5 à 10 mn, et on a maintenu le débit de celui-ci pendant toute l'addition par l'ampoule à brome de la solution suivante :

composé de formule 6 : 111 g

chloroforme R.P. : 650 cc

triéthylamine : 65 cc.

Le temps d'addition a été de 3 à 4 heures. Après addition, on a évaporé la solution au rotavapor. On a repris le résidu par de l'acétone anhydre, filtré le chlorhydrate de triéthylamine insoluble et on l'a bien lavé à l'acétone. On a évaporé la phase acétonique au rotavapor et repris le résidu sirupeux par du benzène anhydre. On a filtré le restant du chlorhydrate de triéthylamine. On a évaporé le benzène et repris le résidu par de l'éthanol absolu bouillant. On a laissé refroidir. Le composé chloroformylé de formule 7a a précipité à froid. On a filtré le précipité à l'éthanol et on l'a lavé puis séché.

On a obtenu 98 g (76 %) du composé de formule 7a dans laquelle $R_3$ est le benzyloxy, $R_{10}$ et $R_{11}$ sont le groupe méthoxy ; point de fusion = 108 °C.

b) étape (7a) : 3-benzyloxy-10,11-diméthoxy-5,6,13,13a-tétrahydro-8H-dibenzo [a, g] quinolizine-8-one (composé de formule 8a)

On a dissous les 98 g du composé chloroformylé précédent dans 500 cc de benzène anhydre (sur Na). On a ajouté 43 g (50 % d'excès) de chlorure de zinc anhydre. On a porté à reflux en agitant vigoureusement pendant 1 h 30. Puis, on a évaporé le benzène au rotavapor. On a repris le résidu par de l'ammoniaque diluée et on l'a extrait au chloroforme. On a lavé la phase chloroformique à l'eau, puis séché sur sulfate de magnésium. On a évaporé au rotavapor le chloroforme. On a repris le résidu par un mélange :

éther : 90 cc

éthanol à 95° : 5 cc

La 8-berbinone a cristallisé très lentement (plusieurs jours). On l'a filtrée, lavée à l'éther, puis séchée.

On a obtenu 55 g (rendement 55 % à 65 %) environ du composé de formule 8a dans laquelle $R_3$ est le groupe benzyloxy et $R_{11}$, $R_{10}$ sont le groupe méthoxy, qui n'est autre qu'un composé de formule II dans laquelle $R_8$ est un oxygène carbonylique, $R_3$, $R_{10}$ et $R_{11}$ étant tels que définis ci-dessus. Point de fusion 144 °C.

c) étape (8a) 3-benzyloxy-10,11-diméthoxy-5,8,13,13a-tétrahydro-6H-dibenzo [a, g] quinolizine (composé de formule II avec

$R_3$ = 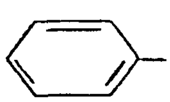 — $CH_2$-O- ; $R_{10}$ et $R_{11}$ = $CH_3$-O- )

Dans un tricol de 3 l, on a introduit 400 cc d'éther anhydre (sur Na) et 11 g d'hydrure de lithium et d'aluminium. On a introduit par une ampoule à brome, goutte à goutte, sous agitation, une solution du composé de formules 8a (55 g) dans 800 $vm^3$ de THF absolu. Après addition, on a laissé en contact une heure et hydrolysé en ajoutant successivement :

11 cc d'eau

11 cc de NaOH à 15 %

33 cc d'eau.

On a filtré les hydroxyles. On les a élués et essorés. On a évaporé le filtrat au rotavapor. On a repris le résidu sirupeux jaune par du méthanol bouillant. Le produit attendu a cristallisé en refroidissant. On a obtenu ainsi 50 g du composé attendu (95 %), point de fusion : 154 °C.

d) 10,11-diméthoxy-3-hydroxy-5,8,13,13a-tétrahydro-6H-dibenzo [a, g]-quinolizine

Dans un ballon de 1 l, on a introduit les 50 g de composé obtenu précédemment et on les a dissous dans 400 $cm^3$ d'acide acétique, on a ajouté 100 $cm^3$ d'acide chlorhydrique concentré et 50 cc d'eau. On a porté à reflux pendant 2 heures. On a évaporé au rotavapor et extrait au chloroforme, puis lavé à l'eau. La phase organique a été séchée sur sulfate de magnésium. On a évaporé au rotavapor et recristallisé le résidu dans de l'alcool à 95°.

On a obtenu 37 g (rendement 95 %), de 3-hydroxy-10,11-diméthoxy-5,8,13,13a-tétrahydro-6H-dibenzo [a, g] quinolizine ayant les caractéristiques indiquées dans l'exemple 1. Ce composé sera dénommé ci-après STB6.

Exemple 4

Préparation de la 3-hydroxy-5,8,13,13a-tétrahydro-6H-dibenzo [a, g] quinolizine (composé de formule II avec $R_3$ = OH, $R_8$, $R_{10}$ et $R_{11}$ = H)

On a utilisé la 3-benzyloxy-phénéthylamine obtenue selon le mode opératoire décrit à l'exemple 1 et on l'a condensée avec l'acide phénylacétique selon le mode opératoire de l'étape (3) par fusion directe vers 200 °C, en quantités sensiblement équimoléculaires. On a obtenu, après déshydratation du sel, l'amide de condensation, c'est-à-dire le N-(3-benzyloxy-phénéthyl) phénacétamide. [rendement : 95 % ; point de fusion : 81-82 °C]

On a fait réagir l'amide ainsi obtenu en solution dans du chloroforme et en présence d'un excès de pentachlorure de phosphore en opérant sensiblement selon le mode opératoire de l'étape (4) décrit à l'exemple 1. On a ainsi obtenu de la 1-benzyl-6-benzyloxy-3,4-dihydroisoquinoléine sous la forme du chlorhydrate [rendement : 68 %, point de fusion 119-120 °C].

On a réduit la dihydroisoquinoléine obtenue précédemment en tétrahydroisoquinoléine par réduction ménagée en présence de zinc dans l'acide acétique dilué sensiblement selon le mode opératoire de l'étape (5) de l'exemple 1. Après extraction, on a obtenu la 1-benzyl-6-benzyloxy-1,2,3,4-tétrahydroisoquinoléine. Rendement 96 %.

La tétrahydroisoquinoléine ainsi obtenue a été condensée avec de l'acide formique sensiblement selon le mode opératoire de l'étape (6) décrit à l'exemple 1 pour obtenir un groupement formyle sur l'azote. On a réalisé cette formylation par fusion à 200 °C du mélange acide formique/tétraisoquinoléine. On a obtenu ainsi la 1-benzyl-6-benzyloxy-2-formyl-tétraisoquinoléine. Rendement 90 %. Point de fusion = 110 °C.

La 2ème cyclisation (étape 7) a été réalisée à partir du composé obtenu ci-dessus dans le chloroforme à reflux en présence de pentachlorure de phosphore. On a procédé comme décrit à l'exemple 1 (étape 7). Il s'est formé une déshydratation interne aboutissant à une structure berbinique mais déshydrogénée aux positions 7 et 8, à savoir au composé ci-après : chlorure de 3-benzyloxy-5,6,13,13a-tétrahydro-dibenzo [a, g] quinolizinium.

On a ensuite réalisé une réduction de la double liaison en 7,8 du chlorure précédent par réduction au zinc en milieu acide chlorhydrique et une hydrolyse du groupement benzylique protecteur de l'hydroxyle phénolique en position 3.

On a obtenu finalement, après recristallisation dans de l'éthanol à 90°, la 3-hydroxy-berbine, c'est-à-dire le composé de formule II avec $R_3$ = OH, $R_8$ = $R_{10}$ = $R_{11}$ = H, qui sera dénommé ci-après « composé STB4 ». (ou composé de formule $II_b$, R = H).

Selon une variante de mise en œuvre, on a opéré selon le même mode opératoire de l'exemple 2 en réduisant de façon ménagée, à l'aide de zinc en milieu acide acétique, la double liaison en 7,8 sans couper le groupe benzylique en position 3 du chlorure obtenu ci-dessus et ensuite on a chauffé le composé résultant (de formule II avec $R_3$ =

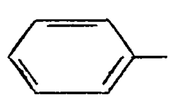 $CH_2$-O-, $R_8$ = $R_{10}$ = $R_{11}$ = H)

en milieu acide chlorhydrique dilué (à 20 %) pour éliminer le groupe benzyle en position 3 et obtenir ainsi la 3-hydroxy-5,8,13,13a-tétrahydro-6H-dibenzo [a, g] quinolizine.

Exemple 5

Préparation de la 3-hydroxy-5,8,13,13a-tétrahydro-6H-dibenzo [a, g]-quinolidzine

On a utilisé comme produit de départ le 3-méthoxy-benzaldéhyde.

Etape (1) :

On a condensé 315 g du 3-méthoxy-benzaldéhyde avec le nitrométhane (450 cc) en milieu acide

acétique anhydre et en présence d'acétate d'ammonium à reflux. On a obtenu du 3-méthoxy-nitrostyrène (302 g) (rendement 73 % ; point de fusion 92 °C).

Etape (2) :

Le nitrostyrène précédent a été réduit avec de l'hydrure de lithium et d'aluminium (116 g) dans le tétrahydrofuranne en 3-méthoxy-phénéthylamine. On a obtenu 154 g de produit.

Etape (3) :

La phénéthylamine (154 g) a ensuite été condensée par fusion à 200 °C (pendant 3 heures environ) avec de l'acide phénylacétique (138,6 g). Il s'est produit une déshydratation du sel et formation du N-(3-méthoxy-phénéthyl)-phénacétamide (rendement 81 %) ; point de fusion 52-53 °C.

Etape (4) :

On a réalisé la 1ère cyclisation en dissolvant l'amide précédent (221 g) dans du chloroforme et en ajoutant du pentachlorure de phosphore (320 g) sensiblement selon le mode opératoire décrit à l'exemple 1 (2 heures à 20 °C). Le chlorhydrate de la dihydroisoquinoléine a précipité dans le milieu. On a obtenu la 1-benzyl-6-méthoxy-3,4-dihydroisoquinoléine sous forme de chlorhydrate (rendement 62,5 %), 147,5 g, point de fusion 165-166 °C.

Etape (5) :

On a réduit au zinc sulfurique la dihydroisoquinoléine précédente en tétrahydrooisoquinoléine à reflux pendant 2 heures. Les sulfates de la tétrahydroisoquinoléine insolubles à froid ont précipité. On a filtré les sulfates et on les a déplacés par la soude diluée ; on a extrait ainsi la 1-benzyl-6-méthoxy-1,2,3,4-tétrahydro-isoquinoléine sous forme de base sirupeuse [126 g ; rendement 75 %].

Etape (6) :

On a condensé sur la tétrahydroisoquinoléine (126 g) du phosgène en milieu basique -triéthylamine) dans le chloroforme. On a obtenu ainsi la 1-benzyl-2-chloroformyl-6-méthoxy-1,2,3,4-tétrahydro-isoquinoléine.

Etape (7) :

Le produit chloroformylé ci-dessus a été cyclisé dans le benzène à reflux en présence de chlorure d'aluminium anhydre comme catalyseur. On a obtenu 115 g de 3-méthoxy-8-berbinone. On a réalisé ainsi la deuxième cyclisation (rendement 83 %-fusion 110 °C).

Etape (8a) :

La 8-berbinone précédente a été réduite par de l'hydrure de lithium et d'aluminium dans le tétrahydrofuranne en 3-méthoxy-berbine (rendement 95 %-fusion 86 °C) [composé de formule II avec $R_3 = CH_3—O—$, $R_8 = R_{10} = R_{11} = H$].

On a hydrolysé le groupe méthoxy en position 3 en hydroxyle phénolique dans de l'acide bromhydrique concentré à reflux pendant 1 h 30. On a obtenu la 3-hydroxy-5,8,13,13a-tétrahydro-6H-dibenzo [a, g] quinolizine (rendement 90 %-fusion du chlorhydrate : décomposition à température supérieure à 200 °C.

## Exemple 6

Préparation des esters du STB6 et STB4 (composés de formule $II_a$ et $II_b$ avec $R_3$ =

R' - C -
‖
O

On a fait réagir le chlorure d'acide de formule

R'-C-Cl
‖
O

avec l'hydroxyle en position 3 du composé de formule $II_a$ ou $II_b$ en présence de triéthylamine dans le chloroforme. Après extraction de l'ester, on le recristallise dans un solvant approprié. On décrit ci-après en détail l'obtention d'un ester du STB6.

a) ester phénylacétique du STB6

Dans un erlenmeyer de 250 $cm^3$ on a dissous du STB6 (5 g) dans 100 $cm^3$ de chloroforme R.P. et 3 $cm^3$ de triéthylamine. On a ajouté goutte à goutte 3 $cm^3$ de chlorure de phénacétoyle. On a laissé le mélange réactionnel sous agitation pendant 15 minutes, puis on a versé dans une ampoule à décanter, lavé à l'eau puis à la soude diluée et à l'eau. On a séché la phase chloroformique sur du sulfate de magnésium. On a filtré et évaporé le filtrat au rotavapor. On a trituré le liquide sirupeux obtenu dans de l'éther. L'ester attendu a cristallisé. On l'a filtré et recristallisé dans de l'éthanol absolu.

On a obtenu ainsi 5,3 g de 3-phénacétyloxy-10,11-diméthoxy-5,8,13,13a-tétrahydro-6H-dibenzo [a, g] quinolizine (rendement 75 %).

| | | |
|---|---|---|
| Rf de la chromatographie en couche mince | $CHCl_3$ 25 cc<br>acétate d'éthyle 25 cc<br>triéthylamine 1 cc | = 0,68 ; point de fusion : 140 °C |

Ce produit sera dénommé ci-après STB 52.

b) Autres esters du STB6 et du STB4

On a opéré sensiblement selon le mode opératoire ci-dessus en utilisant un chlorure différent à chaque fois et on a obtenu les composés indiqués dans le tableau ci-après.

Essais pharmacologiques

Les propriétés pharmacologiques des dérivés STB4 et STB6 et de leurs esters phénylacétiques ont été étudiées sur le système cardio-vasculaire et ont été comparées à celles d'une substance de référence, la Berbine (STB0). La toxicité aiguë des mêmes substances a également été déterminée.

1. Action anti-hypertensive

L'étude a été réalisée sur le rat génétiquement hypertendu (SHR) de souche Okamoto. Les produits ont été administrés en solution dans l'eau, par gavage à l'aide d'une sonde œsophagienne, sous légère anesthésie à l'éther. Des témoins ont reçu un excipient (eau distillée, 5 ml/kg) dans les mêmes conditions. La pression moyenne a été mesurée à l'aide de cathéters implantés à demeure et reliés à une cellule de pression (STATHAM P23DB) et à un appareil amplificateur et enregistreur (SCHWARZER), suivant la méthode décrite par WAEDELE et STOCLET (J. Physiol. Paris, 1973, *66*, 357-366).

(Voir Tableaux pages suivantes)

| Chlorure mis en oeuvre | Composé | Ester obtenu | Dénomination |
|---|---|---|---|
| Chlorure d'acétyle $CH_3-CO-Cl$ | STB 6 | 3-acétyloxy-10,11-diméthoxy-5,8,13,13a-tétrahydro-6H-dibenzo [a, g] quinolizine | STB 53 |
| | STB 4 | 3-acétyloxy-6H- dibenzo [a, g]-5,8,13,13a-tétrahydroquinolizine | STB 91 |
| Chlorure de l'acide propionique | STB 6 | 3-méthylacétyloxy-10,11-diméthoxy-5,8,13,13a-tétrahydro-6H-dibenzo [a, g] quinolizine | STB 50 |
| $CH_3-CH_2-CO-Cl$ | STB 4 | 3-méthylacétyloxy-5,8,13,13a-tétrahydro-6H-dibenzo [a, g] quinolizine | STB 92 |
| Chlorure de l'acide butyrique | STB 6 | 3-éthylacétyloxy-10,11-diméthoxy-5,8,13,13a-tétrahydro-6H-dibenzo [a, g] quinolizine | STB 54 |
| $CH_3CH_2CH_2COCl$ | STB 4 | 3-éthylacétyloxy-5,8,13,13a-tétrahydro-6H-dibenzo [a, g] quinolizine | STB 93 |

| Chlorure mis en oeuvre | Composé | Ester obtenu | Dénomination |
|---|---|---|---|
| Chlorure de l'acide benzoïque 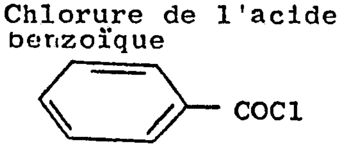 | STB 6 | 3-phénylcarboxy-10,11-diméthoxy-5,8,13,13a-tétrahydro-6H-dibenzo [a, g] quinolizine | STB 51 |
| | STB 4 | 3-phénylcarboxy-5,8,13,13a-tétrahydro-6H-dibenzo [a, g] quinolizine | STB 94 |
| Chlorure de phénacétoyle | STB 4 | 3-phénacétyloxy-5,8,13,13a-tétrahydro-6H-dibenzo-[a, g] quinolizine | STB 69 |
| | STB 6 | 3-phénacétyloxy-10,11-diméthoxy-5,8,13,13a tétrahydro-6H-dibenzo [a, g] quinolizine | STB 52 |

Les résultats obtenus, représentés sur la figure 1, montrent qu'à la dose de 50 mg/kg per os, les cinq produits testés diminuent la pression artérielle du rat SHR vigile de façon très prononcée et prolongée.

La figure 1 indique donc l'évolution, en fonction du temps en heures, de la pression artérielle moyenne (ΔP mmHg en ordonnées) chez le rat SHR vigile auquel on a administré per os, à la dose de 50 mg · $kg^{-1}$, soit STB0 (O—O), soit du STB6 (■—■), soit du STB52 (□—□), soit du STB4 (△—△), soit du STB69 (▲—▲), soit de l'eau distillée à raison de 5 ml · $kg^{-1}$ pour les placebos. La pression moyenne avant traitement étant respectivement (pression moyenne ± erreur type) :

183,3 ± 6,3 mmHg pour le lot STBO (n = 6)
184,2 ± 6,1 mmHg pour le lot STB6 (n = 6)
177,1 ± 6,4 mmHg pour le lot STB52 (n = 7)
187,1 ± 6,3 mmHg pour le lot STB4 (n = 7)
212,5 ± 7,2 mmHg pour le lot STB6 (n = 6)
171,4 ± 5,8 mmHg pour le lot placebo (n = 7)

Chaque point est la moyenne des valeurs obtenues chez les n animaux.

2. Effets sur la réactivité cardio-vasculaire in vivo et sur la résistance vasculaire périphérique.

La réponse vasopressive à l'injection intraveineuse de « Noradrénaline » a été mesurée avant et 60 minutes après administration des substances à tester, chez le rat anesthésié à l'uréthane et traité par un ganglioplégique (suivant la technique décrite par DUPONT et SASSARD, Br. J. Pharmacol. 1974, 50, 185-189). Tous les dérivés ont été administrés comme plus haut par gavage par sonde œsophagienne, à la dose de 50 mg · $kg^{-1}$ dans de l'eau distillée (5 ml · $kg^{-1}$). Les résultats obtenus, représentés sur la figure 2, montrent que, dans ces conditions, tous les composés testés diminuent de façon marquée la réponse pressive à la « Noradrénaline », sans produire de variation significative de la pression de base.

La figure 2 indique donc l'effet d'une injection intraveineuse de Noradrénaline sur la pression artérielle moyenne du rat avant et 1 heure après administration per os de 50 mg · $kg^{-1}$ de dérivé STB dans 5 ml · $kg^{-1}$ d'eau distillée. Le placebo était constitué par administration du même volume d'eau distillée.

Les rats ont été anesthésiés à l'uréthane 1 g · $kg^{-1}$. L'augmentation de pression provoquée par chaque administration de Noradrénaline est mesurée à son maximum.

Les résultats sont la moyenne ± sm de 5 expériences au moins.

Les effets sur le débit cardiaque et la résistance vasculaire périphérique ont été mesurés chez le rat anesthésié par l'« Inactine » (70 mg · $kg^{-1}$), voie intreapéritonéale). Le débit a été mesuré par thermodilution suivant la technique décrite par HONDAS et al. (Path. Biol. 1979, *17* 1069-1078). La pression moyenne a été enregistrée à l'aide d'un cathéter placé dans l'artère fémorale du rat. Les mesures ont été faites avant et entre 45 et 60 minutes après l'administration intra-péritonéale des anti-hypertenseurs. Chaque chiffre expérimental représente la moyenne de trois mesures successives. Les résultats, donnés à titre d'exemple dans le tableau 1, ci-après, en ce qui concerne le STB6, montrent que l'administration de l'anti-hypertenseur provoque une importante chute de pression artérielle imputable à une diminution marquée de la résistance vasculaire périphérique et sans variation significative du débit et du rythme cardiaques, ni du volume d'éjection systolique.

3. Effets sur des artères isolées

L'inhibition des réponses contractiles maximales provoquées par la Phényléphrine (1 × $10^5$ M) a été étudiée sur l'aorte isolée du rat préparée suivant la technique décrite par VALETTE, G. et BA MUOI, N. (Ann. Pharm. franc. 1962, *20*, 557-582) et sur le segment proximal et le segment distal de l'artère caudale suivant la technique décrite par WORCEL, M. (J. Pharmacol. Exp. Therm. 1978, *207*, 210-330). Dans tous les cas, les dérivés STB se sont comportés comme de puissants antagonistes non compétitifs de la Phényléphrine. Leurs concentrations inhibitrices 50 % (ID50) sont indiquées dans le tableau 2. Comme c'est le cas pour d'autres vasodilatateurs (comme l'Hydralazine), l'artère caudale est plus sensible que l'aorte à l'effet inhibiteur des dérivés étudiés.

4. Effets sur l'oreillette isolée de cobaye

Les effets sur le rythme et sur la concentration cardiaques ont été respectivement mis en évidence sur l'oreillette droite battant spontanément et sur l'oreillette gauche stimulée électriquement, suivant la technique décrite par GROSSMAN A., FURCHGOTT R. (J. Pharmacol. Exp. Ther. 1964, *143*, 107-119).

Les résultats consignés dans le tableau 3 font apparaître d'importantes différences entre les dérivés étudiés. Le STB0 (Berbine) déprime fortement la fréquence et la force contractile de base, ainsi que la fréquence maximale en présence d'Isoprotérénol. Les effets dépresseurs sur la fréquence subsistent de façon moins marquée avec le STB6, le STB4 et le STB52, mais ces dérivés ne diminuent pas la force contractile. Bien au contraire, le STB6, et surtout le STB52, augmentent fortement la force contractile (effet inotrope positif). Le STB69 est pratiquement dépourvu d'action sur l'oreillette de cobaye.

5. Toxicité aiguë

La DL 50 a été déterminée chez la souris par la méthode de LITCHFIELD et WILCOXON (J. Pharmacol. 1949 *96*, 99-113). Les dérivés étudiés ont été administrés en solution aqueuse additionnée de 7 % de diméthylformamide, gavage œsophagien de 0,4 ml/20 g) à des souris mâles pesant de 20 à 25 g.

Les résultats consignés dans le tableau 4 montrent que le STB6 est le dérivé le plus toxique et que les esters moins toxiques que les phénols correspondants et que le STB0. Le dérivé STB69 est le moins toxique, mais sa DL50 n'a pas pu être déterminée par voie orale en raison de sa solubilité insuffisante. C'est pourquoi, il a été comparé au STB0 par injection intrapéritonéale (même solution et même volume injectés par voie orale).

Les composés selon l'invention présentent donc des propriétés pharmacologiques qui permettent leur application dans le domaine cardio-vasculaire. Les doses à utiliser dépendent évidemment de l'état du patient et de l'affection cardio-vasculaire à traiter. Toutefois, on indiquera que des doses de 0,30 à 0,43 g par jour sont appropriées.

(Voir Tableaux pages suivantes)

Tableau 1

Effet sur le débit cardiaque (DC, ml, mn · $kg^{-1}$), la pression artérielle moyenne (PA, mmHg), le rythme (en systoles/mm), la résistance vasculaire périphérique totale (RVPt : dynes · mn · $cm^{-5}$ · $kg^{-1}$), le volume d'éjection systolique (ml · mn · $kg^{-1}$) avant et 45 minutes après administration de STB6 par voie i. p. au rat anesthésié à l'Inactine (70 mg · $kg^{-1}$ i. p.)

Chaque valeur est la moyenne de 7 expériences ± erreur type

| | STB6 15 mg.$kg^{-1}$ i.p.n=7 | | | | |
|---|---|---|---|---|---|
| | DC | PA | Rythme | RVPt | Volume éjection systolique |
| Avant | 237,7 ± 12,9 | 150,2 ± 3,9 | 386 ± 11 | 728,9 ± 29,2 | 0,71 ± 0,04 |
| | | ### | | ### | |
| Après | 293,9 ±18,7 n.s. | 89,1 ± 3,3 | 374 ± 12 n.s. | 412,2 ± 17,8 | 0,78 ± 0,035 n.s. |

n. s. = non significatif

###: p. ≤ 0,01

Tableau 2

Inhibition de la contraction d'artères isolées de rat par les dérivés STB. La contraction est suscitée par la phényléphrine $1 \cdot 10^{-5}$ M (aorte), $1 \cdot 10^{-6}$ M (artère caudale proximale) ou $5 \cdot 10^{-6}$ M (artère caudale distale). Les résultats sont exprimés sous forme d'$I_{50}$ et les valeurs sont les moyennes d'au moins 3 expériences ± erreur type.

| SUBSTANCES | EFFET SUR L'AORTE DE RAT | EFFET SUR L'ARTERE CAUDALE DE RAT | |
|---|---|---|---|
| | $I_{50}$ | SEGMENT PROXIMAL | $I_{50}$ SEGMENT DISTAL |
| STBO | $1,3.10^{-5}$ M ± $0,1.10^{-5}$ | $3,0.10^{-7}$ M ± $0,6.10^{-7}$ | $1,3.10^{-6}$ M ± $0,3 10^{-6}$ |
| STB6 | $5.9.10^{-5}$ M ± $0,4.10^{-5}$ | $1,1.10^{-6}$ M ± $0,15.10^{-6}$ | $2,1.10^{-6}$ M ± $0,2.10^{-6}$ |
| STB52 | $5,1.10^{-5}$ M ± $1,2.10^{-5}$ | $2,1.10^{-6}$ M ± $0,3.10^{-6}$ | $4,3.10^{-6}$ M ± $0,57.10^{-6}$ |
| STB4 | $2,6.10^{-5}$ M ± $0,1.10^{-5}$ | $1,0.10^{-6}$ M ± $0,2.10^{-6}$ | $1,9.10^{-6}$ M ± $0,3.10^{-6}$ |
| STB69 | $5,8.10^{-5}$ M ± $2,4.10^{-5}$ | $1,5.10^{-6}$ M ± $0,3.10^{-6}$ | $3,1.10^{-6}$ M ± $1,0.10^{-6}$ |

Tableau 3

Effet sur la fréquence de l'oreillette droite isolée de cobaye et sur la force contractile de l'oreillette gauche isolée de cobaye stimulée électriquement à la fréquence de 2,5 Hz. Les résultats sont la moyenne de 3 expériences ± erreur type.

| Substances | Concentration mole/l | Fréquence | | Force contractile | |
|---|---|---|---|---|---|
| | | Inhibition % du rythme de base | Inhibition % du rythme maximal en présence d'isoprotérénol | Variation % de la force contractile de base | Variation % de la force maximale en présence d'isoprotérénol |
| STBO | $5.10^{-5}$ | 46 ± 5 | 38 ± 6 | - 47 ± 11,5 | - 4 ± 4 |
| STB6 | $5.10^{-5}$ | 8 ± 5 | 21,6 ± 1,2 | + 32 ± 8 | O |
| STB52 | $5.10^{-6}$ | 14 ± 7 | 17 ± 3 | + 90 ± 21 | + 15 ± 1 |
| STB4 | $45.10^{-5}$ | 13,8 ± 5,8 | 28,7 ± 1,8 | O | O |
| STB69 | $5.10^{-5}$ | O | 12,5 ± 2,7 | + 12,6 ± 10 | + 4,3 ± 2,9 |

Tableau 4

Détermination de la DL 50 ($mg \cdot kg^{-1}$) chez la souris (méthode de Litchfield et Wilcoxon). Les valeurs représentent la DL50 et ses limites supérieures et inférieures pour le seuil de probabilité $p = 0{,}05$.

| VOIE ADMINISTRATION | STBO | STB6 | STB52 | STB4 | STB69 |
|---|---|---|---|---|---|
| per os | 464 (426-506) | 275 (252-300) | 550 (468-635) | 464 (438-492) | $>$ 650 |
| ip. | 262 (245-280) | - | - | - | 378 (343-415) |

**Revendications** (pour les Etats contractants : BE, CH, DE, GB, IT, LI, LU, NL, SE)

1. A titre de composés nouveaux, les composés répondant à la formule :

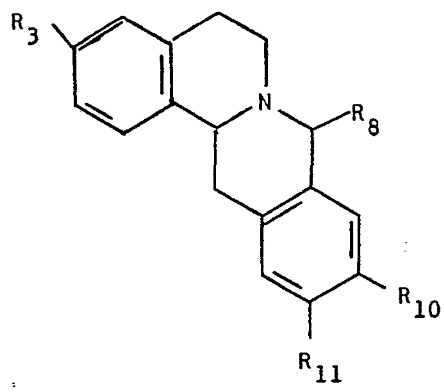

(II)

dans laquelle :

$R_3$ est un radical —OR ou SR, R étant l'hydrogène, un groupe alkyle en $C_1$-$C_{12}$ ou phényle ou phénylalkyle (avec alkyle en $C_1$ à $C_4$) ou un groupe de formule

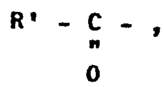

R' étant un groupe alkyle en $C_1$-$C_{12}$ ou phényl, ou phénylalkyle (avec alkyle en $C_1$-$C_4$)

$R_8$ est = 0 ou 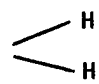

$R_{10}$ et $R_{11}$, identiques ou différents, représentent l'hydrogène ou un groupe alcoxy inférieur contenant 1 à 4 atomes de carbone dans le reste alkyle, à la condition que, lorsque $R_8$ est

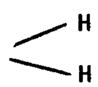

$R_{10}$ et $R_{11}$ sont l'hydrogène ou un groupe alcoxy inférieur, $R_3$ est différent du groupe OR dans lequel R est un groupe alkyle.

2. Composé selon la revendication 1, caractérisés en ce que $R_3$ est le groupe —OR, R étant tel que défini dans la revendication 1, $R_8$ est l'hydrogène et $R_{10}$ et $R_{11}$ sont identiques et représentent l'hydrogène ou le groupe méthoxy.

3. Composés selon l'une des revendications 1 ou 2, caractérisés en ce qu'ils répondent à la formule II dans laquelle $R_8$ est l'hydrogène, $R_{10}$ et $R_{11}$ représentent l'hydrogène et $R_3$ est le groupe OR dans lequel R est l'hydrogène ou un groupe choisi parmi les groupes acétyle, propionyle, butyryle, benzoyle, benzylcarbonyle.

4. Composés selon l'une des revendications 1 ou 2, caractérisés en ce qu'ils répondent à la formule II dans laquelle $R_8$ est l'hydrogène, $R_{10}$ et $R_{11}$ représentent le groupe méthoxy et $R_3$ est le groupe OR dans lequel R est l'hydrogène ou un groupe choisi parmi les groupes acétyle; propionyle, butyryle, benzoyle, benzylcarbonyle.

5. Procédé pour l'obtention des composés selon l'une quelconque des revendications 1 à 4, caractérisé en ce qu'il consiste à :

1. condenser, avec du nitrométhane, le benzaldéhyde substitué en position 3 par un groupe $R_3$ protégé, pour former le nitrostyrène correspondant ;
2. réduire le nitrostyrène obtenu pour former la phénéthylamine correspondante ;
3. condenser la phénéthylamine obtenue avec un sel de l'acide phénylacétique pour former le phénacétamide correspondant ;
4. procéder à une première cyclisation pour obtenir la 3,4-dihydroisoquinoléine correspondante ;
5. réduire la dihydroisoquinoléine en tétrahydroisoquinoléine ;

6. fixer par condensation avec de l'acide formique ou du phosgène du groupe formyle ou chloroformyle sur l'atome d'azote de l'isoquinoléine ;

7. procéder à une seconde cyclisation ;

8. éventuellement, réduire le produit résultant pour former le dérivé de berbine désiré ;

9. éventuellement, éliminer le groupement protecteur en position 3 et

10. éventuellement, estérifier le produit résultant.

6. Procédé selon la revendication 5, caractérisé en ce que la première cyclisation est réalisée à la température ambiante, en présence de pentachlorure de phosphore dans du chloroforme.

7. Procédé selon l'une des revendications 5 ou 6, caractérisé en ce que l'on fixe par condensation un groupe formyle sur l'atome d'azote de l'isoquinoléine et en ce qu'on réalise la seconde cyclisation sur le composé N-formylé ainsi obtenu en présence de pentachlorure.

8. Procédé selon l'une des revendications 5 ou 6, caractérisé en ce que l'on fixe par condensation un groupe chloroformylé sur l'atome d'azote de l'isoquinoléine et en ce qu'on réalise la seconde cyclisation sur le composé N-chloroformyle ainsi obtenu en présencede chlorure d'aluminium anhydre ou de chlorure de zinc anhydre.

9. Procédé selon l'une quelconque des revendications 5 à 8, caractérisé en ce que le composé de départ, à savoir le benzaldéhyde substitué en position 3 par un groupe $R_3$ protégé, est obtenu à partir du benzaldéhyde substitué en 3 par le groupe OH ou SH par condensation avec un chlorure pour bloquer la fonction OH ou SH libre.

10. Procédé selon l'une quelconque des revendications 5 à 9, caractérisé en ce que les étapes 8 et 9 sont réalisées simultanément en présence de zinc en milieu acide fort, tel qu'en milieu acide chlorhydrique ou en milieu acide bromhydrique.

11. Procédé selon l'une quelconque des revendications 5 à 10, caractérisé en ce que l'estérification du composé obtenu après élimination du groupe protecteur en position 3 est réalisée avec un chlorure d'acide en présence d'une base, telle que la triéthylamine.

12. Compositions pharmaceutiques, caractérisées en ce qu'elles contiennent, à titre d'ingrédient actif, un composé selon la revendication 1 en combinaison avec un véhicule approprié.

13. Compositions pharmaceutiques selon la revendication 12, caractérisées en ce qu'elles se présentent sous la forme de compositions injectables ou administrables par voie orale.

14. Composés selon l'une quelconque des revendications 1 à 4, pour l'utilisation dans le traitement des troubles cardio-vasculaires.

**Revendications** (pour l'Etat contractant AT)

1. Procédé de préparation d'un composé de formule

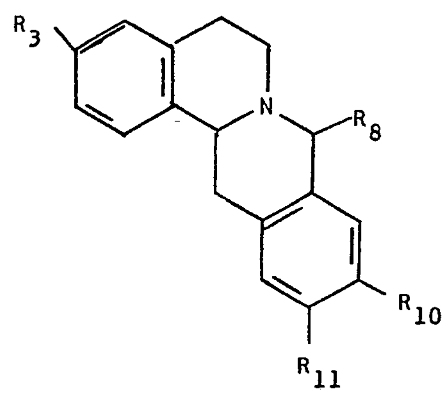

(II)

dans laquelle :

$R_3$ est un radical —OR ou SR, R étant l'hydrogène, un groupe alkyle en $C_1$-$C_{12}$ ou phényle ou phénylalkyle (avec alkyle en $C_1$ à $C_4$) ou un groupe de formule R' C = 0 R' étant un groupe alkyle en $C_1$-$C_{12}$ ou phényl, ou phénylalkyle (avec alkyle en $C_1$-$C_4$)

$R_8$ est = 0 ou 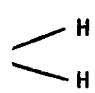

$R_{10}$ et $R_{11}$, identiques ou différents, représentent l'hydrogène ou un groupe alcoxy inférieur contenant 1 à 4 atomes de carbone dans le reste alkyle, à la condition que, lorsque $R_8$ est $H_2$, et $R_{10}$ et $R_{11}$ sont l'hydrogène ou un groupe alcoxy inférieur, $R_3$ est différent du groupe OR dans lequel R est un groupe alkyle caractérisé en ce qu'il consiste à :

1. condenser, avec du nitrométhane, le benzaldéhyde substitué en position 3 par un groupe $R_3$ protégé, pour former le nitrostyrène correspondant ;
2. réduire le nitrostyrène obtenu pour former la phénéthylamine correspondante ;
3. condenser la phénéthylamine obtenue avec un sel de l'acide phénylacétique pour former le phénacétamide correspondant ;
4. procéder à une première cyclisation pour obtenir la 3,4-dihydroisoquinoléine correspondante ;
5. réduire la dihydroisoquinoléine en tétrahydroisoquinoléine ;
6. fixer par condensation avec de l'acide formique ou du phosgène un groupe formyle ou chloroformyle sur l'atome d'azote de l'isoquinoléine ;
7. procéder à une seconde cyclisation ;
8. éventuellement, réduire le produit résultant pour former le dérivé de berbine désiré ;
9. éventuellement, éliminer le groupement protecteur en position 3 et
10. éventuellement, estérifier le produit résultant.

2. Procédé selon la revendication 1, caractérisé en ce que la première cyclisation est réalisée à la température ambiante, en présence de pentachlorure de phosphore dans du chloroforme.

3. Procédé selon l'une des revendications 1 ou 2, caractérisé en ce que l'on fixe par condensation un groupe formyle sur l'atome d'azote de l'isoquinoléine et en ce qu'on réalise la seconde cyclisation sur le composé N-formylé ainsi obtenu en présence de pentachlorure.

4. Procédé selon l'une des revendications 1 ou 2, caractérisé en ce que l'on fixe par condensation un groupe chloroformylé sur l'atome d'azote de l'isoquinoléine et en ce qu'on réalise la seconde cyclisation sur le composé N-chloroformylé ainsi obtenu en présence de chlorure d'aluminium anhydre ou de chlorure de zinc anhydre.

5. Procédé selon l'une quelconque des revendications 1 à 4, caractérisé en ce que le composé de départ, à savoir le benzaldéhyde substitué en position 3 par un groupe $R_3$ protégé, est obtenu à partir du benzaldéhyde substitué en 3 par le groupe OH ou SH par condensation avec un chlorure pour bloquer la fonction OH ou SH libre.

6. Procédé selon l'une quelconque des revendications 1 à 5, caractérisé en ce que les étapes 8 et 9 sont réalisées simultanément en présence de zinc en milieu acide fort, qu'en milieu acide chlorhydrique ou en milieu acide bromhydrique.

7. Procédé selon l'une quelconque des revendications 1 à 6, caractérisé en ce que l'estérification du composé obtenu après élimination du groupe protecteur en position 3 est réalisée avec un chlorure d'acide en présence d'une base, telle que la triéthylamine.

8. Procédé selon l'une quelconque des revendications précédentes caractérisé en ce que dans la formule II $R_3$ est le groupe OR, R étant tel que défini à la revendication 1, $R_8$ est H et $R_{10}$ et $R_{11}$ sont identiques et représentent OH ou $OCH_3$.

9. Procédé selon l'une quelconque des revendications 1 à 8, caractérisé en ce que dans la formule II, $R_8$, $R_{10}$ et $R_{11}$ sont H et $R_3$ est le groupe OR dans lequel R est H ou un groupe choisi parmi les groupes acétyle, propionyle, butyryle, benzole ou benzycarbonyle.

10. Procédé selon l'une quelconque des revendications 1 à 8, caractérisé en ce que dans la formule II $R_8$ est H, $R_{10}$ et $R_{11}$ sont $OCH_3$ et $R_3$ est OR, dans lequel R est choisi parmi H ou les groupes acétyle, propionyle, butyryle, benzole, benzylcarbonyle.

11. Procédé de préparation de compositions pharmaceutiques, caractérisé en ce qu'on mélange un produit de formule II dans laquelle $R_3$, $R_8$, $R_{10}$ et $R_{11}$ sont comme définis dans la revendication 1 avec un véhicule pharmaceutiquement acceptable.

12. Procédé selon la revendication 11, caractérisé en ce que le produit de formule II est tel que $R_{10} = R_{11} = OCH_3$ et $R_3$ est OR, dans lequel R est choisi parmi H ou les groupes acétyle, propionyle, butyryle, benzole, benzylcarbonyle.

**Claims** (for the Contracting States : BE, CH, DE, GB, IT, LI LU, NL, SE)

1. By way of new compounds, the compounds having the formula :

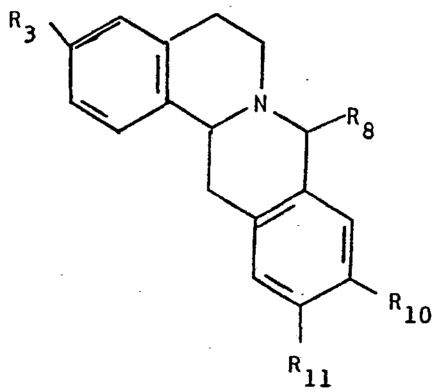

(II)

wherein :

$R_3$ is a —OR or —SR group, R being hydrogen, $C_1$-$C_{12}$ alkyl or phenyl or phenylalkyl (with $C_1$ to $C_4$ alkyl) or a group of formula

$$R' - \underset{\underset{O}{\|}}{C} - ,$$

R' being $C_1$-$C_{12}$ alkyl, or phenyl or phenylalkyl (with $C_1$-$C_4$ alkyl) ;

$R_8$ is = 0 or 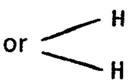

$R_{10}$ and $R_{11}$, which may be identical or different, are hydrogen or lower $C_1$-$C_4$ alkoxy provided that when $R_8$ is

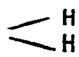

$R_{10}$ and $R_{11}$ are hydrogen or lower alkoxy, $R_3$ is different from OR in which R is alkyl.

2. Compounds according to claim 1, characterised in that $R_3$ is —OR, R being as defined in claim 1, $R_8$ is hydrogen and $R_{10}$ and $R_{11}$ are identical and represents hydrogen or methoxy.

3. Compounds according to one of claims 1 or 2, characterised in that they have formula II wherein $R_8$ is hydrogen, $R_{10}$ and $R_{11}$ represents hydrogen and $R_3$ is OR wherein R is hydrogen or a group selected from acetyl, propionyl, butyryl, benzol and benzylcarbonyl.

4. Compounds according to one of claims 1 or 2, characterised in that they have formula II wherein $R_8$ is hydrogen, $R_{10}$ and $R_{11}$ represents methoxy and $R_3$ is OR wherein R is hydrogen or a group selected from acetyl, propionyl, butyryl, benzoyl and benzylcarbonyl.

5. Process for obtaining compounds according to any one of claims 1 to 4, characterised in that it consists in :

1. condensing, with nitromethane, benzaldehyde substituted in the 3-position by a protected $R_3$ group to form corresponding nitrostyrene ;
2. reducing nitrostyrene obtained to form the corresponding phenethylamine ;
3. condensing phenethylamine obtained with a salt of phenylacetic acid to form the corresponding phenacetamide ;
4. performing a first cyclisation to obtain the corresponding 3,4-dihydroisoquinoline ;
5. reducing the dihydroisoquinoline to tetrahydroisoquinoline ;
6. fixing formyl or chloroformyl group on the nitrogen atom of the isoquinoline by reaction with formic acid or phosgene :
7. performing a second cyclisation ;
8. optionally, reducing the resulting product to form the desired berbine derivative ;
9. optionally, removing the protecting group from the 3-position, and
10. optionally, converting the resulting product to an ester.

6. Process according to claim 5, characterised in that the first cyclisation is carried out at room temperature in the presence of phosphorous pentachloride in chloroform.

7. Process according to one of claims 5 or 6, characterised in that formyl group is fixed on the nitrogen atom of the isoquinoline, and in that the second cyclisation is carried out on the N-formylated compound thus obtained, in the presence of pentachloride.

8. Process according to one of claims 5 or 6, characterised in that chloroformyl group is attached on the nitrogen atom of the isoquinoline, and in that the second cyclisation is carried out on the N-chloroformyl compound thus obtained in the presence of anhydrous aluminium chloride or of anhydrous zinc chloride.

9. Process according to any one of claims 5 to 8, characterised in that the starting compound, namely benzaldehyde substituted in the 3-position by a protected $R_3$ group, is obtained from benzaldehyde substituted in the 3-position by OH or SH by reaction with a chloride in order to block the free OH or SH group.

10. Process according to any one of claims 5 to 9, characterised in that steps 8 and 9 are carried out simultaneously in the presence of zinc in strong acid medium, such as hydrochloric acid medium or hydrobromic acid medium.

11. Process according to any one of claims 5 to 10, characterised in that the conversion to an ester of the compound obtained after removal of the protecting group from the 3-position is carried out with an acid chloride in the presence of a base, such as triethylamine.

12. Pharmaceutical compositions characterised in that they comprise as active ingredient, a compound according to claim 1 together with a suitable vehicle.

13. Pharmaceutical compositions according to claim 12, characterised in that they are in forms which can be injected or administered orally.

14. Compounds according to any one of claims 1 to 4, for use in the treatment of cardiovascular disorders.

**Claims** (for the Contracting State AT)

1. Process for the obtention of a compound having formula :

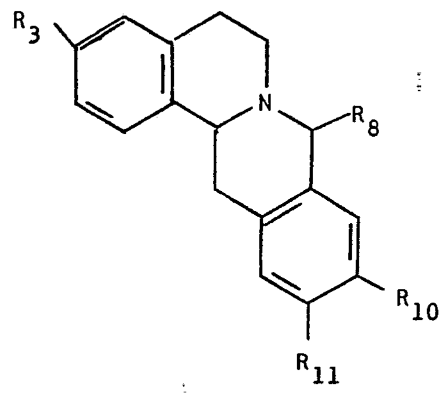

(II)

wherein :

$R_3$ is —OR or SR group, R being hydrogen, $C_1$-$C_{12}$ alkyl or phenyl or phenylalkyl (with $C_1$ to $C_4$ alkyl) or a group of formula R'—C = 0, R' being $C_1$-$C_{12}$ alkyl or a phenyl or phenylalkyl (with $C_1$-$C_4$ alkyl) ;

$R_8$ is = 0 or 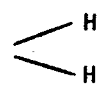

$R_{10}$ and $R_{11}$, which may be identical or different, represent hydrogen or lower $C_1$-$C_4$ alkoxy group provided that when $R_8$ is $H_2$ and $R_{10}$ and $R_{11}$ are hydrogen or a lower alkoxy group, $R_3$ is different from OR in which R is an alkyl group,

characterized in that it consists in :

1. condensing, with nitromethane, benzaldehyde substituted in the 3-position by a protected $R_3$ group, to form the corresponding nitrostyrene ;
2. reducing the nitrostyrene obtained to form the corresponding phenethylamine ;
3. condensing the phenethylamine obtained with a salt of phenylacetic acid to form the corresponding phenacetamide ;
4. performing a first cyclisation to obtain the corresponding 3,4-dihydroisoquinoline ;
5. reducing the dihydroisoquinoline to tetrahydroisoquinoline ;
6. fixing formyl or chloroformyl group on the nitrogen atom of the isoquinoline by reaction with formic acid or phosgene ;
7. performing a second cyclisation ;
8. optionally, reducing the resulting product to form the desired berbine derivative ;
9. optionally, removing the protecting group from the 3-position, and
10. optionally, converting the resulting product to an ester.

2. Process according to claim 1, characterised in that the first cyclisation is carried out at room temperature in the presence of the presence of phosphorous pentachloride in chloroform.

3. Process according to one of claims 1 or 2, characterised in that formyl is fixed on the nitrogen atom of the isoquinoline, and in that the second cyclisation is carried out on the N-formylated compound thus obtained in the presence of pentachloride.

4. Process according to one of claims 1 or 2, characterised in that a chloroformyl group is fixed on the nitrogen atom of the isoquinoline, and in that the second cyclisation is carried out on the N-chloroformyl compound thus obtained in the presence of anhydrous aluminium chloride or anhydrous zinc chloride.

5. Process according to any one of claims 1 to 4, characterised in that the starting compound, namely benzaldehyde substituted in the 3-position by a protected $R_3$ group, is obtained from benzaldehyde substituted in the 3-position by OH or SH by reaction with a chloride in order to block the free OH or SH group.

6. Process according to anyone of claims 1 to 5, characterised in that steps 8 and 9 are carried out

simultaneously in the presence of zinc in a strong acid medium, such as a hydrochloric acid medium or a hydrobromic acid medium.

7. Process according to any one of claims 1 to 6, characterised in that the conversion to an ester of the compound obtained after removal of the protecting group from the 3-position is carried out with an acid chloride in the presence of a base, such as triethylamine.

8. Process according to any one of the preceding claims, characterised in that, in the formula II, $R_3$ is OR, R being as defined in claim 1, $R_8$ is H ans $R_{10}$ and $R_{11}$ are identical and represents OH or $OCH_3$.

9. Process according to any one of claims 1 to 8, characterised in that, in the formula II, $R_8$, $R_{10}$ and $R_{11}$ are H and $R_3$ is OR, wherein R is H or a group selected from acetyl, propionyl, butyryl, benzoyl or benzylcarbonyl.

10. Process according to any one of claims 1 to 8, characterised in that in the formula II, $R_8$ is H, $R_{10}$ and $R_{11}$ are $OCH_3$ and $R_3$ is OR, wherein R is selected from H or acetyl, propionyl, butyryl, benzoyl and benzylcarbonyl groups.

11. Process for the obtention of pharmaceutical compositions, characterised in that a product of formula II, in which $R_3$, $R_8$, $R_{10}$ and $R_{11}$ are as defined in claim 1, is mixed with a pharmaceutically acceptable vehicle.

12. Process according to claim 11, characterised in that the product of formula II is such that $R_{10} = R_{11} = OCH_3$ and $R_3$ is OR, wherein R is selected from H or acetyl, propionyl, butyryl, benzoyl and benzylcarbonyl groups.

**Ansprüche** (für die Vertragsstaaten : BE, CH, DE, GB, IT, LI, LU, NL, SE)

1. Als neue Verbindungen die Verbindungen der Formel

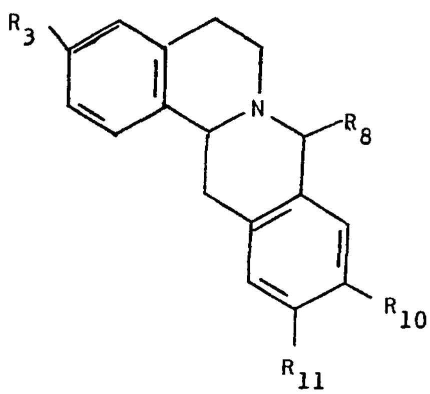

(II)

in der

$R_3$ ein Rest der Formel —OR oder SR, worin R Wasserstoff, ein $C_1$-$C_{12}$-Alkylrest oder ein Phenyl- oder Phenylalkylrest (mit 1 bis 4 C-Atomen im Alkylrest) ist, oder eine Gruppe der Formel

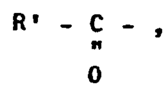

ist, worin R' ein $C_1$-$C_{12}$-Alkylrest oder ein Phenyl- oder Phenylalkylrest (mit 1 bis 4 C-Atomen im Alkylrest) ist,

$R_8$ für = 0 oder 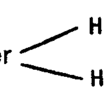

steht ;

$R_{10}$ und $R_{11}$, die gleich oder verschieden sind, Wasserstoff oder einen niederen Alkoxyrest mit 1 bis 4 C-Atomen im Alkylrest darstellen, mit dem Vorbehalt, daß, wenn $R_8$ für

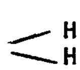

steht $R_{10}$ und $R_{11}$ Wasserstoff oder ein niederer Alkoxyrest sind, $R_3$ verschieden vom Rest OR ist, in dem R ein Alkylrest ist.

2. Verbindungen nach Anspruch 1, dadurch gekennzeichnet, daß $R_3$ die Gruppe —OR ist, in der R die in Anspruch 1 genannte Bedeutung hat, $R_8$ Wasserstoff ist und $R_{10}$ und $R_{11}$ gleich sind und Wasserstoff oder die Methoxygruppe darstellen.

3. Verbindungen nach einem der Ansprüche 1 oder 2, dadurch gekennzeichnet, daß sie die Formel II haben, in der $R_8$ Wasserstoff ist, $R_{10}$ und $R_{11}$ Wasserstoff sind und $R_3$ die Gruppe OR ist, in der R

Wasserstoff oder eine Gruppe ist, die aus Acetyl-, Propionyl-, Butyryl-, Benzoyl- und Benzylcarbonylgruppen ausgewählt ist.

4. Verbindungen nach einem der Ansprüche 1 oder 2, dadurch gekennzeichnet, daß sie die Formel II haben, in der $R_8$ Wasserstoff ist, $R_{10}$ und $R_{11}$ die Methoxygruppe darstellen und $R_3$ die Gruppe OR ist, in der R Wasserstoff oder eine Gruppe ist, die aus Acetyl-, Propionyl-, Butyryl-, Benzoyl- und Benzylcarbonylgruppen ausgewählt ist.

5. Verfahren zur Herstellung der Verbindungen nach irgendeinem der Ansprüche 1 bis 4, dadurch gekennzeichnet, daß man

1. in 3-Stellung durch einen geschützten Rest $R_3$ substituierten Benzaldehyd mit Nitromethan kondensiert und hierdurch das entsprechende Nitrostyrol bildet,
2. das erhaltene Nitrostyrol reduziert und hierdurch das entsprechende Phenethylamin bildet,
3. das erhaltene Phenethylamin mit einem Salz der Phenylessigsäure kondensiert und hierdurch das entsprechende Phenacetamid bildet,
4. eine erste Cyclisierung vornimmt und hierbei das entsprechende 3,4-Dihydroisochinolin erhält,
5. das Dihydroisochinolin zum Tetrahydroisochinolin reduziert,
6. durch Kondensation mit Ameisensäure oder Phosgen eine Formyl- oder Chloroformylgruppe an das Stickstoffatom des Isochinolins bindet,
7. eine zweite Cyclisierung vornimmt,
8. das erhaltene Produkt gegebenenfalls reduziert und hierdurch das gewünschte Berbinderivat bildet,
9. die Schutzgruppe in 3-Stellung gegebenenfalls entfernt und
10. das hierbei erhaltene Produkt gegebenenfalls verestert.

6. Verfahren nach Anspruch 5, dadurch gekennzeichnet, daß man die erste Cyclisierung bei Umgebungstemperatur in Gegenwart von Phosphorpentachlorid in Chloroform durchführt.

7. Verfahren nach einem der Ansprüche 5 oder 6, dadurch gekennzeichnet, daß man durch Kondensation eine Formylgruppe am Stickstoffatom des Isochinolins bindet, und daß man die zweite Cyclisierung der so erhaltenen N-Formylverbindung in Gegenwart von Pentachlorid durchführt.

8. Verfahren nach einem der Ansprüche 5 oder 6, dadurch gekennzeichnet, daß man durch Kondensation eine chloroformylgruppe an das Stickstoffatom des Isochinolins bindet, und daß man die zweite Cyclisierung der so erhaltenen N-chloroformylierten Verbindung in Gegenwart von wasserfreiem Aluminiumchlorid oder von wasserfreiem Zinkchlorid durchführt.

9. Verfahren nach irgendeinem der Ansprüche 5 bis 8, dadurch gekennzeichnet, daß die Ausgangsverbindung, d. h. der in 3-Stellung durch einen geschützten Rest $R_3$ substituierte Benzaldehyd, aus Benzaldehyd, der in 3-Stellung durch eine OH- oder SH-Gruppe substituiert ist, durch Kondensation mit einem Chlorid, um die freie OH- oder SH-Funktion zu blockieren, erhalten wird.

10. Verfahren nach irgendeinem der Ansprüche 5 bis 9, dadurch gekennzeichnet, daß die Stufen 8 und 9 gleichzeitig in Gegenwart von Zink in stark saurem Medium, z. B. in Salzsäuremedium oder Bromwasserstoffsäuremedium, durchgeführt werden.

11. Verfahren nach irgendeinem der Ansprüche 5 bis 10, dadurch gekennzeichnet, daß die Veresterung der nach Entfernung der Schutzgruppe in 3-Stellung erhaltenen Verbindung mit einem Säurechlorid in Gegenwart einer Base, z. B. Triethylamin, durchgeführt wird.

12. Pharmazeutische Zubereitungen, dadurch gekennzeichnet, daß sie als Wirkstoff eine Verbindung nach Anspruch 1 in Kombination mit einem geeigneten Träger enthalten.

13. Pharmazeutische Zubereitungen nach Anspruch 12, dadurch gekennzeichnet, daß sie die Form von injizierbaren oder oral verabreichbaren Zusammensetzungen haben.

14. Verbindungen nach irgendeinem der Ansprüche 1 bis 4 für die Verwendung zur Behandlung kardiovaskulärer Störungen.

**Ansprüche** (für den Vertragsstaat AT)

1. Verfahren zur Herstellung einer Verbindung der Formel

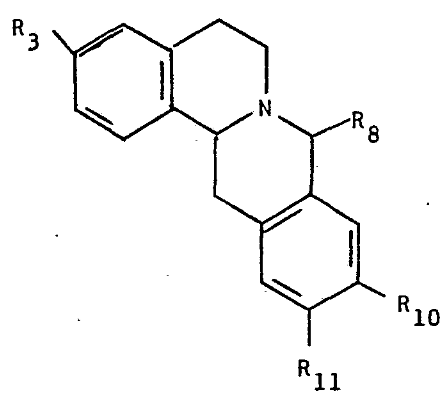

(II)

in der

$R_3$ ein Rest der Formel —OR oder SR, worin R Wasserstoff, ein $C_1$-$C_{12}$-Alkylrest oder ein Phenyloder Phenylalkylrest (mit 1 bis 4 C-Atomen im Alkylrest) ist, oder ein Rest der Formel R'C=O ist, worin R' ein $C_1$-$C_{12}$-Alkylrest oder ein Phenylrest oder Phenylalkylrest (mit 1 bis 4 C-Atomen im Alkylrest) ist,

$R_8$ für = 0 oder 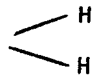

steht,

$R_{10}$ und $R_{11}$ gleich oder verschieden sind und Wasserstoff oder einen niederen Alkoxyrest mit 1 bis 4 C-Atomen im Alkylrest darstellen, mit dem Vorbehalt, daß, wenn $R_8$ für $H_2$ und $R_{10}$ und $R_{11}$ für Wasserstoff oder einen niederen Alkoxyrest stehen, $R_3$ verschieden vom Rest OR ist, in dem R ein Alkylrest ist, dadurch gekennzeichnet, daß man

1. in 3-Stellung durch einen geschützten Rest $R_3$ substituierten Benzaldehyd mit Nitromethan kondensiert und hierdurch das entsprechende Nitrostyrol bildet,

2. das erhaltene Nitrostyrol reduziert und hierdurch das entsprechende Phenethylamin bildet,

3. das erhaltene Phenethylamin mit einem Salz der Phenylessigsäure kondensiert und hierdurch das entsprechende Phenacetamid bildet,

4. eine erste Cyclisierung vornimmt und hierbei das entsprechende 3,4-Dihydroisochinolin erhält,

5. das Dihydroisochinolin zum Tetrahydroisochinolin reduziert,

6. durch Kondensation mit Ameisensäure oder Phosgen eine Formyl- oder Chloroformylgruppe an das Stickstoffatom des Isochinolins bindet,

7. eine zweite Cyclisierung vornimmt,

8. das erhaltene Produkt gegebenenfalls reduziert und hierdurch das gewünschte Berbinderivat bildet,

9. die Schutzgruppe in 3-Stellung gegebenenfalls entfernt und

10. das hierbei erhaltene Produkt gegebenenfalls verestert.

2. Verfahren nach Anspruch 1, dadurch gekennzeichnet, daß die erste Cyclisierung bei Umgebungstemperatur in Gegenwart von Phosphorpentachlorid in Chloroform durchgeführt wird.

3. Verfahren nach einem der Ansprüche 1 oder 2, dadurch gekennzeichnet, daß man durch Kondensation eine Formylgruppe an das Stickstoffatom des Isochinolins bindet, und daß man die zweite Cyclisierung der so erhaltenen N-Formylverbindung in Gegenwart von Pentachlorid durchführt.

4. Verfahren nach einem der Ansprüche 1 oder 2, dadurch gekennzeichnet, daß man durch Kondensation eine Chloroformylgruppe an das Stickstoffatom des Isochinolins bindet, und daß man die zweite Cyclisierung der so erhaltenen N-Chloroformylverbindung in Gegenwart von wasserfreiem Aluminiumchlorid oder von wasserfreiem Zinkchlorid durchführt.

5. Verfahren nach irgendeinem der Ansprüche 1 bis 4, dadurch gekennzeichnet, daß die Ausgangsverbindung, d. h. der in 3-Stellung durch einen geschützten Rest $R_3$ substituierte Benzaldehyd, aus Benzaldehyd, der in 3-Stellung durch eine OH- oder SH-Gruppe substituiert ist, durch Kondensation mit einem Chlorid, um die freie OH- oder SH-Funktion zu blockieren, erhalten wird.

6. Verfahren nach irgendeinem der Ansprüche 1 bis 5, dadurch gekennzeichnet, daß die Stufen 8 und 9 gleichzeitig in Gegenwart von Zink in stark saurem Medium, z. B. in Salzsäuremedium oder Bromwasserstoffsäuremedium, durchgeführt werden.

7. Verfahren nach irgendeinem der Ansprüche 1 bis 6, dadurch gekennzeichnet, daß die Veresterung der nach Entfernung der Schutzgruppe in 3-Stellung erhaltenen Verbindung mit einem Säurechlorid in Gegenwart eine Base, z. B. Triethylamin, durchgeführt wird.

8. Verfahren nach irgendeinem der vorstehenden Ansprüche, dadurch gekennzeichnet, daß in der Formel II $R_3$ die Gruppe OR, ist, in der R die in Anspruch 1 genannte Bedeutung hat, $R_8$ H ist und $R_{10}$ und $R_{11}$ gleich sind und OH oder $OCH_3$ darstellen.

9. Verfahren nach irgendeinem der Ansprüche 1 bis 8, dadurch gekennzeichnet, daß in der Formel II $R_8$, $R_{10}$ und $R_{11}$ H sind und $R_3$ die Gruppe OR ist, in der R für H oder eine Gruppe steht, die aus Acetyl-, Propionyl-, Butyryl-, Benzoyl- oder Benzylcarbonylgruppen ausgewählt ist.

10. Verfahren nach irgendeinem der Ansprüche 1 bis 8, dadurch gekennzeichnet, daß in der Formel II $R_8$ für H steht, $R_{10}$ und $R_{11}$ für $OCH_3$ stehen und $R_3$ für OR steht, worin R aus H oder Acetyl-, Propionyl-, Butyryl-, Benzoyl und Benzylcarbonylgruppen ausgewählt ist.

11. Verfahren zur Herstellung von pharmazeutischen Zubereitungen, dadurch gekennzeichnet, daß man ein Produkt der Formel II, in der $R_3$, $R_8$, $R_{10}$ und $R_{11}$ die in Anspruch 1 genannten Bedeutungen haben, mit einem pharmazeutisch annehmbaren Träger mischt.

12. Verfahren nach Anspruch 11, dadurch gekennzeichnet, daß das Produkt der Formel II derart ist, daß $R_{10}$ = $R_{11}$ = $OCH_3$ und $R_3$ für OR steht, worin R aus H oder Acetyl-, Propionyl-, Butyryl-, Benzoyl- und Benzylcarbonylgruppen ausgewählt ist.

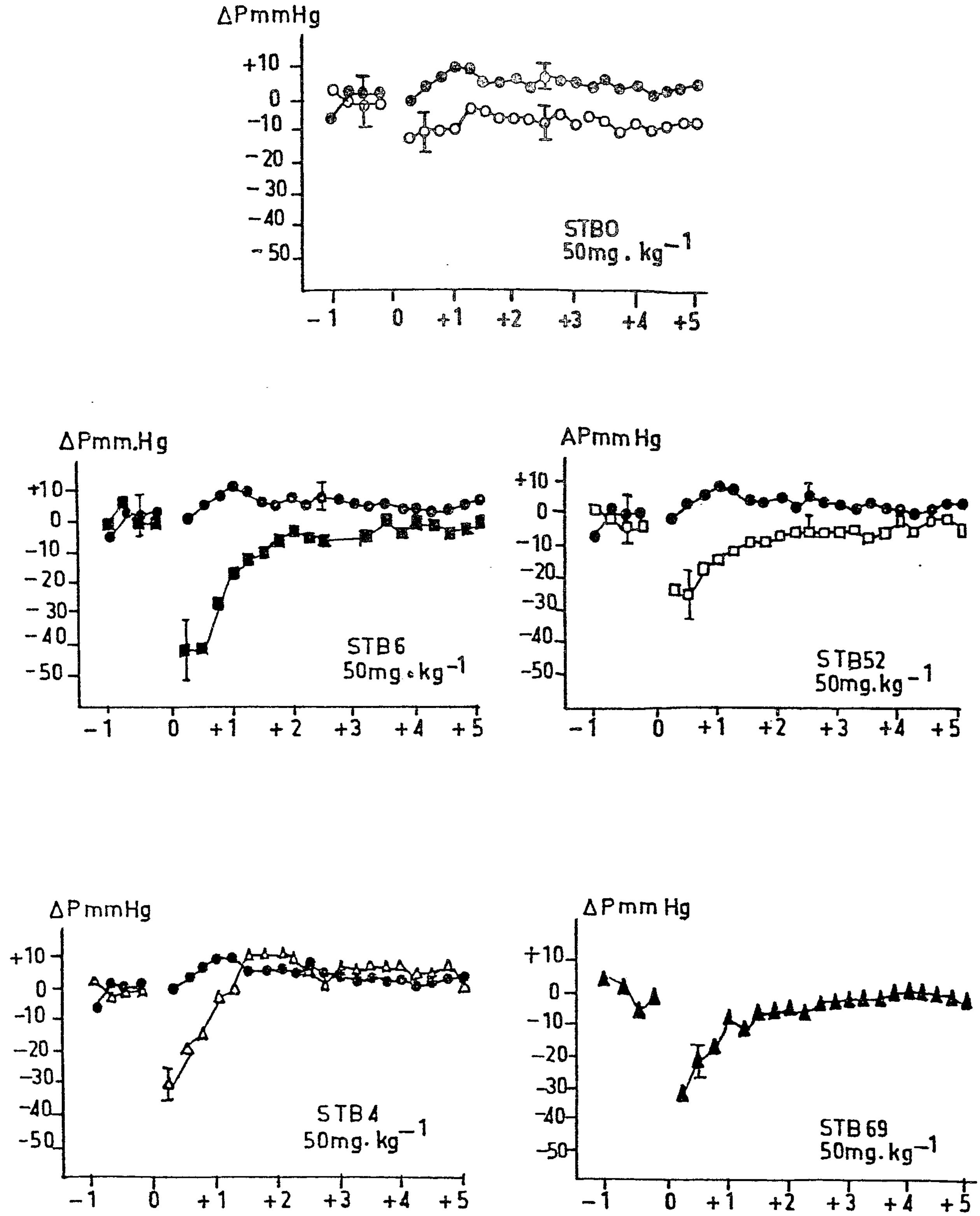
ΔPmmHg
STB0
50mg . kg$^{-1}$
ΔPmm.Hg
STB6
50mg.kg$^{-1}$
ΔPmm Hg
STB52
50mg.kg$^{-1}$
ΔPmmHg
STB4
50mg. kg$^{-1}$
ΔPmm Hg
STB69
50mg. kg$^{-1}$

FIG.1

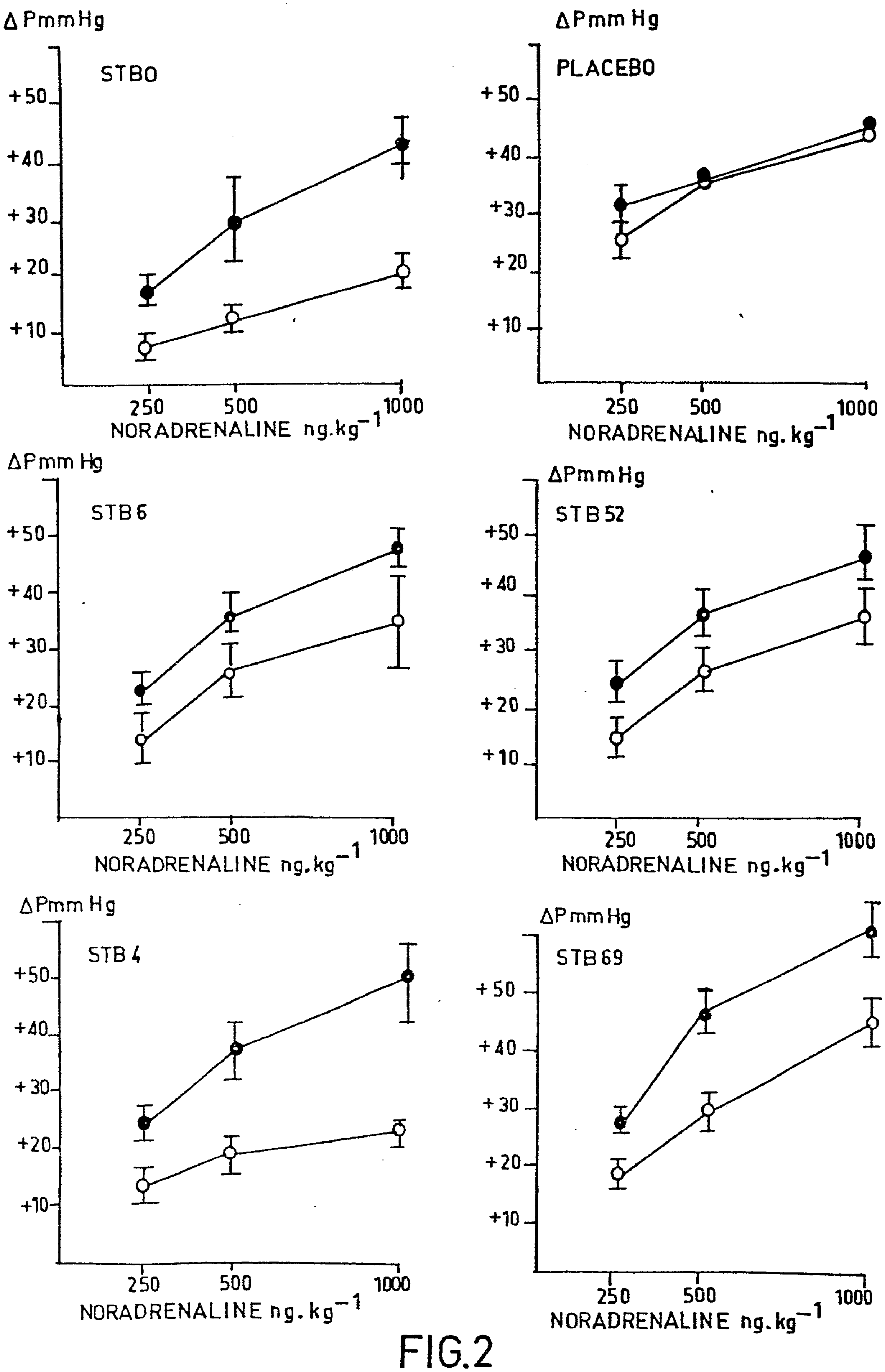
ΔPmmHg
STBO
+50
+40
+30
+20
+10
250
500
1000
NORADRENALINE ng.kg$^{-1}$
ΔPmm Hg
PLACEBO
+50
+40
+30
+20
+10
250
500
1000
NORADRENALINE ng.kg$^{-1}$
ΔPmm Hg
STB 6
+50
+40
+30
+20
+10
250
500
1000
NORADRENALINE ng.kg$^{-1}$
ΔPmmHg
STB 52
+50
+40
+30
+20
+10
250
500
1000
NORADRENALINE ng.kg$^{-1}$
ΔPmm Hg
STB 4
+50
+40
+30
+20
+10
250
500
1000
NORADRENALINE ng.kg$^{-1}$
ΔPmmHg
STB 69
+50
+40
+30
+20
+10
250
500
1000
NORADRENALINE ng.kg$^{-1}$

FIG.2